# EUROPÄISCHE PATENTSCHRIFT

(11) **EP 1 435 877 B1**
(45) Veröffentlichungstag und Bekanntmachung des Hinweises auf die Patenterteilung: **15.04.2009**
(21) Anmeldenummer: 02779166.4
(22) Anmeldetag: 15.10.2002
(51) Int. Cl.: A61F 2/06, A61L 27/00

(54) **BESCHICHTUNG VON STENTS ZUR VERHINDERUNG VON RESTENOSE**
COATING OF STENTS FOR PREVENTING RESTENOSIS
REVETEMENT DE STENTS EMPECHANT LA RESTENOSE

(30) Priorität: 15.10.2001 DE 10150340; 26.11.2001 DE 10157610
(43) Veröffentlichungstag der Anmeldung: 14.07.2004
(73) Patentinhaber: Hemoteq AG, 52146 Würselen (DE)
(72) Erfinder: HORRES, Roland, 52223 Stolberg (DE); HOFFMANN, Erika, 52249 Eschweiler (DE); HOFFMANN, Michael, 52249 Eschweiler (DE); FAUST, Volker, 52080 Aachen (DE); DI BIASE, Donato, 52080 Aachen (DE)
(74) Vertreter: Arth, Hans-Lothar
(86) Internationale Anmeldenummer: PCT/DE2002/003941
(87) Internationale Veröffentlichungsnummer: WO 2003/034944

(56) Entgegenhaltungen:
- EP-A- 0 623 354
- WO-A-02/13883
- WO-A-91/12779
- US-A- 5 380 299
- US-A- 5 464 450
- US-A- 5 980 551
- US-B1- 6 299 604
- US-B1- 6 355 055

## Beschreibung

Die Erfindung betrifft Stents gemäß Anspruch 1 und Verfahren zur Herstellung dieser Stents.

In den letzten Jahren hat das Einsetzen von Stents bei der Ballondilatation von verschlossenen Blutgefäßen immer weitere Verbreitung gefunden. Obwohl Stents das Risiko eines emeuten Gefäßverschlusses verkleinern, sind sie bisher nicht in der Lage, solche Restenosen vollständig zu verhindern.

Eine genaue begriffliche Beschreibung der Restenose ist in der Fachliteratur nicht aufzufinden. Die am häufigsten verwendete morphologische Definition der Restenose ist diejenige, die nach erfolgreicher PTA (perkutane transluminale Angioplastie) die Restenose als eine Reduktion des Gefäßdurchmessers auf weniger als 50% des normalen festlegt. Hierbei handelt es sich um einen empirisch festgelegten Wert, dessen hämodynamische Bedeutung und Beziehung zur klinischen Symptomatik einer soliden wissenschaftlichen Basis entbehrt. In der Praxis wird häufig die klinische Verschlechterung eines Patienten als Zeichen einer Restenose des vormals behandelten Gefäßabschnitts angesehen.

Für die durch den Stent verursachte Restenose gibt es zwei verschiedene Ursachen:
a.) In der ersten Zeit nach der Implantation ist die Oberfläche des Stents dem Blut direkt ausgesetzt und es kann aufgrund der nun vorhandenen Fremdoberfläche zu einer akuten Thrombose kommen, die das Blutgefäß wieder verschließt
b.) Mit Implantation des Stent werden Gefäßverletzungen verursacht, die neben der in oben genannten Thrombose, ebenfalls Entzündungsreaktionen hervorrufen, die für den Heilungsprozeß in den ersten sieben Tagen eine entscheidende Rolle spielen. Die hierbei ablaufenden Prozesse sind unter anderem mit der Ausschüttung von Wachstumsfaktoren verbunden, womit eine verstärkte Proliferation der glatten Muskelzellen eingeleitet wird und damit schon kurzfristig zu einem erneuten Verschluß des Gefäßes aufgrund unkontrollierten Wachstums führen.
c.) Nach einigen Wochen beginnt der Stent in das Gewebe des Blutgefäßes einzuwachsen. Das heißt, daß der Stent vollständig von glatten Muskelzellen umhüllt wird und keinen Kontakt mehr zum Blut hat. Diese Vemarbung kann zu stark ausgeprägt sein (Neointimahyperplasie) und dazu führen, daß nicht nur die Stentoberfläche bedeckt, sondern der ganze Innenraum des Stents verschlossen wird.

Man hat vergeblich versucht, das Problem der Restenose durch die Beschichtung der Stents mit Heparin zu lösen (J. Whörle et al, European Heart Journal 2001, 22, 1808-1816). Heparin adressiert als Antikoagulanz jedoch nur die erstgenannte Ursache und kann darüber hinaus nur in Lösung seine volle Wirkung entfalten. Dieses erste Problem läßt sich mittlerweile medikamentös durch die Gabe von Antikoagulantien fast vollständig vermeiden. Das zweite und dritte Problem versucht man zur Zeit zu lösen, indem man das Wachstum der glatten Muskelzellen lokal am Stent hemmt. Das wird z.B. mit radioaktiven Stents oder mit Stents versucht, welche pharmazeutische Wirkstoffe enthalten.

So offenbart US-A-5 891 108 beispielsweise einen hohl ausgeformten Stent, welcher in seinem Inneren pharmazeutische Wirkstoffe enthalten kann, welche durch eine Vielzahl von Öffnungen im Stent freigesetzt werden. EP-A-1 127 582 beschreibt hingegen einen Stent, der auf seiner Oberfläche Einbuchtungen von 0,1 - 1 mm Tiefe und 7 - 15 mm Länge aufweist, welche zur Aufnahme eines Wirkstoffes geeignet sind. Diese Wirkstoffreservoire setzen, vergleichbar der Öffnungen in dem hohlen Stent, den enthaltenen pharmazeutischen Wirkstoff punktuell in hoher Konzentration und über einen relativ langen Zeitraum frei, was aber dazu führt, daß die glatten Muskelzellen nicht mehr oder nur sehr verzögert in der Lage sind, den Stent zu umhüllen. Daher ist der Stent viel länger dem Blut ausgesetzt, was wieder vermehrt zu Gefäßverschlüssen durch Thrombosen führt (Liistro F., Colombo A., Late acute thrombosis after Paclitaxel eluting stent implantation. Heart 2001, 86, 262-264).
Ein Lösungsversuch für dieses Problem stellt die Phosphorylcholinbeschichtung von Biocompatlbles (WO 0101957) dar, indem hier Phosphorylcholin, eine Zellmembrankomponente der Erythrocyten, als Bestandteil der aufgebrachten nicht bioabbaubaren Polymerschicht auf dem Stent eine nichtthrombogene Oberfläche erzeugen soll. Dabei wird der Wirkstoff abhängig vom Molekulargewicht von dieser polymerhaltigen Phosphorylcholinschicht absorbiert oder auf der Oberfläche adsorbiert. US-B-6 299 604 beschreibt Stents mit mehrlagigen Beschichtungen.

Aufgabe der vorliegenden Erfindung ist es, Stents bereitzustellen, welche ein kontinuierliches kontrolliertes Einwachsen des Stents in die Gefäßwand erlauben einerseits durch Unterdrückung der cellulären Reaktionen in den ersten Tagen und Wochen nach Implantation mit Hilfe der ausgewählten Wirkstoffe und Wirkstoffkombinationen und andererseits durch die Bereitstellung einer athrombogenen bzw. inerten bzw. biokompatiblen Oberfläche gewährleistet, dass mit Abklingen der Wirkstoffeinflusses und Abbau der Matrix keine Reaktionen auf die bestehende Fremdoberfläche mehr einsetzen, die langzeitlich ebenfalls zu einem Wiederverschluß des Blutgefäßes führen können.
Diese Aufgabe wird durch die technische Lehre der unabhängigen Ansprüche der vorliegenden Erfindung gelöst. Weitere vorteilhafte Ausgestaltungen der Erfindung ergeben sich aus den abhängigen Ansprüchen, der Beschreibung sowie den Beispielen.

Die erfindungsgemäßen Stents sind mit einer hämokompatiblen Schicht überzogen und besitzen eine oder mehrere weitere Schichten, die mindestens einen antiproliferativen oder/und entzündungshemmenden und bei Bedarf auch antithrombotischen Wirkstoff enthalten.
Die hämokompatible Beschichtung eines Stents sorgt für die notwendige Blutverträglichkeit und der Wirkstoff (oder Wirkstoffkombination), der über die Gesamtoberfläche des Stents gleichmäßig verteilt ist, bewirkt, daß der Bewuchs der Stentoberfläche mit Zellen, insbesondere glatte Muskel- und Endothelzellen, in einer kontrollierten Weise abläuft. Es findet somit keine rasche Besiedelung und Überwucherung der Stentoberfläche mit Zellen statt, was zu einer Restenose führen könnte, wohingegen der Bewuchs der Stentoberfläche mit Zellen aber auch nicht durch eine hohe Medikamentenkonzentration vollkommen unterbunden wird, was die Gefahr einer Thrombose mit sich bringt.

Somit gewährleistet die Einbettung von Wirkstoffen, dass der Wirkstoff oder die Wirkstoffkombination, kovalent oder/und adhäsiv an die darunterliegende Schicht gebunden oder/und kovalent oder/und adhäsiv in die Schicht eingelagert, kontinuierlich und in geringen Dosen freigesetzt wird, so dass nicht die Besiedelung der Stentoberfläche mit Zellen unterbunden, jedoch eine Überbesiedelung verhindert wird. Diese Kombination beider Effekte verleiht dem erfindungsgemäßen Stent die Fähigkeit, schnell in die Gefäßwand einzuwachsen und vermindert sowohl das Risiko einer Restenose als auch das Risiko einer Thrombose. Die Freisetzung des oder der Wirkstoffe erstreckt sich über einen Zeitraum von 1 bis 12 Monaten, bevorzugt über 1-2 Monate nach Implantation.

Als Wirkstoffe werden antiproliferative Substanzen, antiphlogistische als auch antithrombotische Stoffe eingesetzt. Als antiproliferative Wirkstoffe werden bevorzugt Cytostatika, Makrolidantibiotika, und/oder Statine eingesetzt. Anwendbare antiproliferative Wirkstoffe sind Sirolimus (Rapamycin), Everolimus, Somatostatin, Tacrolimus, Roxithromycin, Dunaimycin, Ascomycin, Bafilomycin, Erythromycin, Midecamycin, Josamycin, Concanamycin, Clarithromycin, Troleandomycin, Folimycin, Cerivastatin, Simvastatin, Lovastatin, Fluvastatin, Rosuvastatin, Atorvastatin, Pravastatin, Pitavastatin, Vinblastin, Vincristin, Vindesin, Vinorelbin, Etobosid, Teniposid, Nimustin, Carmustin, Lomustin, Cyclophosphamid, 4-Hydroxyoxycyclophosphamid Estramustin, Melphalan, Betulinsäure, Camptothecin, Lapachol, β-Lapachon,Podophyllotoxin, Betulin, Tropfosfamid, Podophyllsäure-2-ethylhydrazid, Ifosfamid, Chlorambucil, Bendamustin, Dacarbazin, Busulfan, Procarbazin, Treosulfan, Tremozolomid, Thiotepa, Daunorubicin, Doxorubicin, Aclarubicin, Epirubicin, Mitoxantron, Idarubicin, Bleomycin, Mitomycin, Dactinomycin, Methotrexat, Fludarabin, Fludarabin-5'-dihydrogenphosphat, Mofebutazon, Acemetacin, Diclofenac, Lonazolac Dapson, o-Carbamoylphenoxyessigsäure, Lidocain, Ketoprofen, Mefenaminsäure, Piroxicam, Meloxicam, Chloroquinphosphat, Penicillamin, Hydroxychloroquin, Auranofin, Natriumaurothiomalat Oxaceprol, Celecoxib, β-Sitosterin, Ademetionin, Myrtecain, Polidocanol, Nonivamid, Levomenthol, Benzocain, Aescin, Cladribin, Mercaptopurin, Thioguanin, Cytarabin, Fluorouracil, Gemcitabin, Capecitabin, Docetaxel, Carboplatin, Cisplatin, Oxaliplatin. Amsacrin, Irinotecan, Topotecan, Hydroxycarbamid, Miltefosin, Pentostatin. Aldesleukin, Tretinoin, Asparaginase, Pegasparase, Anastrozol, Exemestan, Letrozol, Formestan, Aminoglutethemid, Adriamycin, Azithromycin, Spiramycin, Cepharantin, SMC-Proliferation-Inhibitor-2w, Epothilone A und B, Mitoxanthrone, Azathioprin, Mycophenolatmofetil, c-myc-Antisense, b-myc-Antisense Selectin (Cytokinantagonist) CETP-Inhibitor, Cadherine, Cytokininhibitoren, COX-2-Inhibitor, NFkB, Angiopeptin, Ciprofloxacin, Camptothecin, Fluroblastin, monoklonale Antikörper, die die Muskelzellproliferation hemmen, bFGF-Antagonisten" Probucol, Prostaglandine, Colchicin, NO-Donoren wie Pentaerythrityltetranitrat und Syndnoelmine, S-Nftrosoderivate, Tamoxifen, Staurosporin, ß-Estradiol, α-Estradiol, Estron, Estriol, Ethinylestradiol, Fosfestrol, Medroxyprogesteron, Estradiolcypionate, Estradiolbenzoate, Tranilast, Kamebakaurin und andere Terpenoide, die in der Krebstherapie eingesetzt werden, Verapamil, Tyrosin-Kinase-Inhibitoren (Tyrphostine), Cyclosporin A, Paclitaxel und dessen Derivate (6-α-Hydroxy-Paclitaxel, Baccatin, Taxotere u.a.), synthetisch hergestellte als auch aus nativen Quellen gewonnene macrocyclische Oligomere des Kohlensuboxids (MCS) und seine Derivate, Molgramostim (rhuGM-CSF), Peginterferon α-2b, Lanograstim (r-HuG-CSF), Filgrastim, Macrogol, Dacarbazin, Basiliximab, Daclizumab, Ellipticin, D-24851 (Calbiochem), Colcemid, Cytochalasin A-E, Indanocine, Nocadazole, S 100 Protein, Bacitracin Vitronectin-Rezeptor Antagonisten, Azelastin, Guanidylcyclase-Stimulator Gewebsinhibitor der Metallproteinase-1 und 2, freie Nukleinsäuren, Nukleinsäuren in Virenüberträger inkorporiert, DNA- und RNA-Fragmente. Plaminogen-Aktivator Inhibitor-1. Plasminogen-Aktivator Inhibitor-2, Antisense Oligonucleotide, VEGF-inhibitoren, IGF-1 genannt. Aus der Gruppe der Antibiotika finden des weiteren Cefadroxil, Cefazolin, Cefaclor. Cefotixin Tobramycin, Gentamycin Anwendung. Positiven Einfluß auf die postoperative Phase haben auch Penicilline wie Dicloxacillin, Oxacillin, Sulfonamide, Metronidazol, Antithrombotika wie Argatroban, Asprin, Abciximab, synthetisches Antithrombin. Bivalirudin, Coumadin, Enoxoparin, Hemoparin, Gewebe-Plasminogen-Aktivator, Gpllb/llla-Plättchenmembran-rezeptor, Faktor Xₐ-inhibitor. Antikörper, Heparin, Hirudin, r-Hirudin, PPACK, Protamin, Prourokinase, Streptokinase, Warfarin, Urokinase, Vasodilatoren wie Dipyramidol, Trapidil, Nitroprusside, PDGF-Antagonisten wie Triazolopyrimidin und Seramin, ACE-Inhibitoren wie Captopril, Cilazapril, Lisinopril, Enalapril, Losartan, Thioproteaseinhibitoren. Caspaseinhibitoren. Apoptoseinhibitoren, Apoptose-regulatoren wie p65 NF-kB und Bcl-xL-Antisense-Oligonukleotiden und Prostacyclin, Vapiprost, α,β- und γ-lnterferon, Histaminantagonisten, Serotoninblocker, , Halofuginon, Nifedipin, Tocopherol, Tranirast, Molsidomin, Teepolyphenole, Epiratechingallat, Epigallocatechingallat Boswellinsäuren und ihre Derivate, Leflunomid, Anakinra, Etanercept. Sulfasalazin,, Etoposid, Dicloxacyllin, Tetracyclin. Triamcinolon, Mutamycin, Procainimid, Retinolsäure, Quinidin, Disopyrimid, Flecainid, Propafenon, Sotolol, Amidoron. Weitere Wirkstoffe sind Steroide (Hydrocortison, Betamethason, Dexamethason), nichtsteroidale Substanzen (NSAIDS) wie Fenoporfen, Ibuprofen, Indomethacin, Naproxen, Phenylbutazon und andere. Antivirale Agentien wie Acyclovir, Ganciclovir und Zidovudin sind ebenfalls einsetzbar. Verschiedene Antimykotika finden Anwendung in diesem Bereich. Beispiele sind Clotrimazol, Flucytosin, Griseofulvin, Ketoconazol, Miconazol, Nystatin, Terbinafin. Antiprozoale Agentien wie Chloroquin, Mefloquin, Quinin sind gleichermassen wirksame Agentien, des weiteren natürliche Terpenoide wie Hippocaesculin, Baningtogenol-C21-angelat, 14-Dehydroagrostistachin, Agroskerin, Agrostistachin, 17-Hydroxyagrostistachin, Ovatodiolide, 4,7-Oxycycloanisomelsäure, Baccharinoide B1, B2, B3, Tubeimosid, Bruceanole A,B,C, Bruceantinoside C, Yadanzioside N, und P, Isadeoxyelephantopin, Tomenphantopin A und B, Coronarin A,B,C und D, Ursolsäure, Hyptatsäure A, Zeorin, Iso-Iridogermanal, Maytenfoliol, Effusantin A, Excisanin A und B, Longikaurin B, Sculponeatin C, Kamebaunin, Leukamenin A und B, 13,18-Dehydro-6-alpha-Senecioyloxychaparrin, 1,11-Dimethoxycanthin-6-on, 1-Hydroxy-11-Methoxycanthin-6-on, Scopolectin, Taxamairin A und B, Regenilol, Triptolid, des weiteren Cymarin, Apocymarin, Aristolochsäure, Anopterin, Hydroxyanopterin, Anemonin, Protoanemonin, Berberin, Cheliburinchlorid, Cictoxin, Sinococulin, Bombrestatin A und B, Cudraisoflavon A, Curcumin, Dihydronitidin, Nitidinchlorid, 12-beta-Hydroxypregnadien-4,16-dien 3,20-dion, Bilobol, Ginkgol, Ginkgolsäure, Helenalin, Indicin, Indicin-N-oxid. Lasiocarpin, Inotodiol, Glykosid 1a, Podophyllotoxin, Justicidin A und B, Larreatin, Malloterin, Mallotochromanol, Isobutyrylmallotochromanol, Maquirosid A, Marchantin A, Maytansin, Lycoridicin, Margetin, Pancratistatin, Liriodenin, Oxoushinsunin, Aristolactam-All, Bisparthenolidin, Periplocosid A, Ghalakinosid, Ursolsäure, Deoxypsorospermin, Psycorubin, Ricin A, Sanguinarin, Manwuweizsäure, Methylsorbifolin, Sphatheliachromen, Stizophyllin, Mansonin, Streblosid, Akagerin, Dihydrousambaraensin, Hydroxyusambarin, Strychnopentamin, Strychnophyllin, Usambarin, Usambarensin, Berberin, Liriodenin, Oxoushinsunin, Daphnoretin, Lariciresinol, Methoxylariciresinol, Syringaresinol, Umbelliferon, Afromoson, Acetylvismion B, Desacetylvismion A, Vismion A und B, weitere natürliche Terpenoide wie Hippocaesculin, 14-Dehydroagrostistachin, Agroskerin, Agrostistachin, 17-Hydroxyagrostistachin, Ovatodiolide, 4,7-Oxycycloanisomelsäure, Yadanzioside N und P, Isodeoxyelephantopin, Tomenphantopin A und B, Coronarin A,B,C und D, Ursolsäure, Hyptatsäure A, Zeorin, Iso-Iridogermanal, Maytenfoliol, Effusantin A, Excisanin A und B, Longikaurin B, Sculponeatin.

Die Wirkstoffe werden einzeln oder kombiniert in gleicher oder unterschiedlicher Konzentration eingesetzt. Besonders bevorzugt sind Wirkstoffe, welche neben ihrer antiproliferativen Wirkung auch noch immunsuppressive Eigenschaften aufweisen. Zu derartigen Wirkstoffen zählen Erythromycin, Midecamycin, Tacrolimus, Sirolimus, Paclitaxel und Josamycin. Zudem bevorzugt ist eine Kombination von mehreren antiproliferativ wirkenden Substanzen oder von antiproliferativen Wirkstoffen mit immunsuppressiven Wirkstoffen. Bevorzugt für die vorliegende Erfindung sind Tacrolimus, Paclitaxel und Derivate, Trapidil, α-und ß-Estradiol, Macrocyclisches Kohlensuboxid (MCS) und deren Derivate und Sirolimus.

Der Wirkstoff ist bevorzugt in einer pharmazeutisch aktiven Konzentration von 0,001-10 mg pro cm² Stentoberfläche enthalten. Weitere Wirkstoffe können in ähnlicher Konzentration in derselben oder in weiteren Schichten enthalten sein.

Die den Stent unmittelbar bedeckende hämokompatible Schicht besteht bevorzugt aus synthetisch hergestellten Heparinderivaten unterschiedlicher Sulfatierungsgrade und Acteylierungsgrade im Molekulargewichtsbereich von des für die antithrombotische Wirkung verantwortlichen Pentasaccharides bis zum Standard molekulargewicht des käuflichen Heparins, Heparansulfaten und dessen Derivate, Oligo- und Polysaccharide der Erythrocytenglycolcalix, die in perfekter Weise die athrombogene Oberfläche der Erythrocyten wiedergeben, da hier im Gegensatz zum Phosphorylcholin der tatsächliche Kontakt von Blut und Erythrocytenoberfläche stattfindet, vollständig desulfatiertem und N-reacetyliertem Heparin, desulfatiertem und N-reacetyliertem Heparin, N-carboxymethyliertem und/oder partiell N-acetyliertem Chitosan und/oder Mischungen dieser Substanzen. Diese Stents mit einer hämokompatiblen, Beschichtung werden hergestellt, indem man herkömmliche in der Regel unbeschichtete Stents bereitstellt und bevorzugt kovalent eine hämokompatible Schicht aufbringt, welche nach Wirkstofffreigabe und damit nach Abklingen des Wirkstoffeinflusses und Abbau der Matrix die Oberfläche des Implantates permanent maskiert.
Die herkömmlichen Stents, welche gemäß der erfindungsgemäßen Verfahren beschichtet werden können, bestehen aus Edelstahl, Nitinol oder anderen Metallen und Legierungen oder aus synthetischen Polymeren.

Die bevorzugte Ausführungsform der erfindungsgemäßen Stents weist eine Beschichtung auf, welche aus mindestens zwei Schichten besteht. Auch Multischichtsysteme finden Anwendung. Bei derartigen Mehrschichtsystemen wird als erste Schicht diejenige bezeichnet, welche direkt auf den Stent aufgebracht ist.
Als zweite Schicht wird diejenige Schicht bezeichnet, welche auf die erste Schicht aufgebracht wird, usw..

Gemäß der Zweischichtausführung besteht die erste Schicht aus einer hämokompatiblen Schicht, welche im wesentlichen vollständig durch eine bioabbaubare Schicht überzogen ist, die mindestens einen antiproliferativen, antiphlogistischen und/oder antithrombotischen Wirkstoff, kovalent oder/und adhäsiv gebunden, enthält. Ebenso werden auch Wirkstoff-Kombinationen eingesetzt, die sich in ihrer Wirkung gegenseitig unterstützen und ergänzen. Als bioabbaubare Substanzen, welche für die äußere Schicht eingesetzt werden können, zählen Polyvalerolactone, Poly-ε-Decalactone, Polylactonsäure, Polyglycolsäure Polylactide. Polyglycolide, Copolymere der Polylactide und Polyglycolide, Poly-ε-caprolacton, Polyhydroxybuttersäure, Polyhydroxybutyrate, Polyhydroxyvalerate, Polyhydroxybutyrate-co-valerate, Poly(1,4-dioxan-2,3-dione), Poly(1,3-dioxan-2-one), Poly-p-dioxanone, Polyan-hydride wie Polymaleinsäureanhydride, Polyhydroxymethacrylate, Fibrin, Polycyanoacrylate, Polycaprolactondimethylacrylate, Poly-b-Maleinsäure Polycaprolactonbutyl-acrylate, Multiblockpolymere wie z.B. aus Oligocaprolactondiole und Oligodioxanondiole, Polyetherestermultiblockpolymere wie z.B. PEG und Polybutylenterephtalat. Polypivotolactone, Polyglycolsäuretrimethylcarbonate, Polycaprolactonglycolide, Poly-g-ethylglutamat, Poly(DTH-Iminocarbonat), Poly(DTE-co-DT-carbonat). Poly(Bisphenol A-iminocarbonat), Polyorthoester, Polyglycolsäuretrimethyl-carbonate, Polytrimethylcarbonate Polyiminocarbonate, Poly(N-vinyl)-Pyrolidon, Polyvinylalkohole, Polyesteramide, glycolierte Polyester, Polyphosphoester, Polyphosphazene, Poly[p-carboxyphenoxy)propan], Polyhydroxypentansäure, Polyanhydride, Polyethylenoxid-propylenoxid, weiche Polyurethane, Polyurethane mit Aminosäureresten im Backbone, Polyetherester wie das Polyethylanoxid, Polyalkenoxalate, Polyorthoester sowie deren Copolymere, Lipide, Carrageenane, Fibrinogen, Stärke, Kollagen, protein-basierende Polymere, Polyaminosäuren, synthetische Polyaminosäuren, Zein, modifiziertes Zein, Polyhydroxyalkanoate, Pectinsäure, Actinsäure, modifiziertes und unmodifiziertes Fibrin und Casein, Carboxymethylsulfat, Albumin, desweiteren Hyaluronsäure, Heparansulfat, Heparin, Chondroitinsulfat, Dextran, b-Cyctodextrine, und Copolymere mit PEG und Polypropylenglycol, Gummi arabicum, Guar, Gelatine, Collagen, Collagen-N-Hydroxysuccinimid, Lipide, Phospholipide, Modifikationen und Copolymere und/oder Mischungen der vorgenannten Substanzen eingesetzt.

Die wirkstoffenthaltende Schicht bzw. Schichten wird langsam durch Blutbestandteile abgebaut, so daß der Wirkstoff entsprechend der Geschwindigkeit des Abbaus der äußeren Schicht freigesetzt wird oder sich entsprechend seines Elutionsverhalten aus der Matrix löst, Die erste hämokompatible Schicht gewährleistet die notwendige Blutverträglichkeit des Stents, sobald die bioabbaubare Schicht degradiert worden ist. Durch diesen biologischen Abbau der äußeren Schicht und die dementsprechende Wirkstofffreisetzung wird ein Anwachsen von Zellen nur für eine gewisse Zeit stark reduziert und ein gezieltes kontrolliertes Anwachsen dort ermöglicht, wo die äußere Schicht bereits weitgehend abgebaut worden ist. Der biologische Abbau der äußeren Schicht erstreckt sich vorteilhafterweise über 1 bis 36 Monate, bevorzugt aber 1-6 Monate, besonders bevorzugt 1-2 Monate. Es hat sich gezeigt, daß bei derartigen Stents Restenosen verhindert oder zumindest sehr stark reduziert werden. In dieser Zeit spielen sich die wichtigen Heilungsprozesse ab. Schlussendlich bleibt die hämokompatible Schicht als athrombogene Oberfläche erhalten und maskiert die Fremdoberfläche derart, dass weiterhin keine lebensbedrohliche Reaktion eintreten kann.

Derartige Stents lassen sich durch ein Verfahren zur hämokompatiblen. Beschichtung von Stents herstellen, dem folgendes Prinzip zugrundeliegt:
a. Bereitstellen eines unbeschichteten Stents
b. Aufbringen einer bevorzugt kovalent gebundenen hämokompatiblen Schicht
c. im wesentlichen vollständiges Überziehen der hämokompatiblen Schicht im Tauch- oder Sprühverfahren mit mindestens einen Wirkstoff. oder
c. im wesentlichen vollständiges Überziehen oder/und unvollständiges Überziehen der hämokompatiblen Schicht im Tauch- oder Sprühverfahren mit mindestens einer bioabbaubaren oder/und biostabilen Schicht, welche mindestens einen Wirkstoff enthält oder/und den Wirkstoff selbst darstellt.

Das Beschichtungsprinzip bietet eine große Variationsbreite bezüglich der gestellten Anforderungen an den Wirkstoff und lässt sich in verschiedene Beschichtungstypen aufteilen, die ebenfalls untereinander kombinierbar sind.

Beschichtungsprinzip 1 :
a.) Bereitstellen eines unbeschichteten Stents
b.) Aufbringen einer hämokompatiblen Schicht;
c.) Aufbringen eines Wirkstoffes oder einer Wirkstoffkombination auf die hämokompatible Schicht ohne Matrix.
d.) Aufbringen eines Wirkstoffes oder einer Wirkstoffkombination auf die hämokompatible Schicht ohne Matrix und im wesentlichen vollständiges oder/und unvollständiges Überziehen der Schichten mit einem bioabbaubaren oder/und biostabilen Material zur Diffusionskontrolle.

Beschichtungsprinzip II :
a.) Bereitstellen eines unbeschichteten Stents
b.) Aufbringen einer hämokompatiblen Schicht;
c.) im wesentlichen vollständiges Überziehen oder/und unvollständiges Überziehen der hämokompatiblen Schicht mit mindestens einer bioabbaubaren oder/und bistabilen Schicht, welche mindestens einen Wirkstoff kovalent an die hämokompatible Schicht gebunden oder/und adhäsiv enthält
d.) im wesentlichen vollständiges Überziehen der hämokompatiblen Schicht mit mindestens einer bioabbaubaren oder/und biostabilen Schicht, welche mindestens einen Wirkstoff kovalent an die Matrix gebunden oder/und adhäsiv enthält und eine weitere, die darunterliegende Schicht vollständig oder/und teilweise überdeckende bioabbaubare oder/und biostabile Schicht ohne Wirkstoff als Diffusionsbarriere.

Beschichtungsprinzip III :
a.) Bereitstellen eines unbeschichteten Stents
b.) Aufbringen einer hämokompatiblen Schicht;
c.) im wesentlichen vollständiges Überziehen der hämokompatiblen Schicht mit mindestens einer bioabbaubaren oder/und biostabilen Schicht, welche mindestens einen Wirkstoff kovalent gebunden oder/und adhäsiv enthält.
d.) Aufbringen eines Wirkstoffes oder einer Wirkstoffkombination kovalent an die darunterliegende Schicht gebunden oder/und adhäsiv
e.) im wesentlichen vollständiges Überziehen der hämokompatiblen Schicht mit mindestens einer bioabbaubaren oder/und biostabilen Schicht, welche mindestens einen Wirkstoff, kovalent gebunden oder/und adhäsiv, enthält, Aufbringen eines Wirkstoffes oder einer Wirkstoffkombination und eine weitere, die darunterliegende Schicht vollständig oder/und teilweise überdeckende bioabbaubare oder/und biostabile Schicht ohne Wirkstoff als Diffusionsbarriere

Beschichtungsprinzip IV :
a.) Bereitstellen eines unbeschichteten Stents
b.) Aufbringen einer hämokompatiblen Schicht:
c.) im wesentlichen vollständiges oder/und unvollständiges Überziehen der hämokompatiblen Schicht mit mindestens zwei bioabbaubaren oder/und biostabilen Schichten, welche mindestens, kovalent oder/und adhäsiv, einen Wirkstoff in unterschiedlicher Konzentration je Schicht enthalten.
d.) im wesentlichen vollständiges oder/und unvollständiges Überziehen der hämokompatiblen Schicht mit mindestens zwei bioabbaubaren oder/und biostabilen Schichten, welche mindestens einen Wirkstoff kovalent oder/und adhäsiv in unterschiedlicher Konzentration je Schicht enthalten und mindestens eine weitere, die darunterliegende Schicht vollständig oder/und teilweise überdeckende, bioabbaubare oder/und biostabile Schicht ohne Wirkstoff als Diffusionsbarriere.
e.) im wesentlichen vollständiges oder/und unvollständiges Überziehen der hämokompatiblen Schicht mit mindestens einer bioabbaubaren oder/und biostabilen Schicht, welche mindestens einen Wirkstoff oder/und mindestens einen weiteren Wirkstoff derselben Gruppe oder aus einer anderen Gruppe mit komplementären Eigenschaften in gleicher oder unterschiedlicher Konzentrationen in kovalenter oder/und adhäsiver Form enthält.
f.) im wesentlichen vollständiges oder/und unvollständiges Überziehen der hämokompatiblen Schicht mit mindestens zwei bioabbaubaren oder/und biostabilen Schichten, welche mindestens einen Wirkstoff oder/und mindestens einen weiteren Wirkstoff derselben Gruppe oder aus einer anderen Gruppe mit komplementären Eigenschaften in gleicher oder unterschiedlicher Konzentrationen enthalten und mindestens eine weitere, die darunterliegende Schicht vollständig oder/und teilweise überdeckende, bioabbaubare oder/und biostabile Schicht ohne Wirkstoff als Diffusionsbarriere.
g.) im wesentlichen vollständiges Überziehen der hämokompatiblen Schicht mit mindestens zwei bioabbaubaren oder/und biostabllen Schichten, welche mindestens einen Wirkstoffen, kovalent oder/und adhäsiv, in gleicher oder/und unterschiedlichen Konzentrationen enthalten und eine weitere, die darunterliegende Schicht vollständig oder auch nur teilweise überdeckende, bioabbaubare oder/und biostabile Schicht ohne Wirkstoff als Diffusionsbarriere und einer diese Schicht überziehenden Wirkstoffschicht bestehend aus mindestens einem Wirkstoff, kovalent oder/und adhäsiv gebunden, ohne Träger.

Eine weitere vorteilhafte Ausführungsform stellt ein Stent mit einer mindestens dreilagigen Beschichtung dar, wobei die erste Schicht die Oberfläche des Stents mit der hämokompatiblen Schicht bedeckt, die zweite Schicht den Wirkstoff enthält und nicht bioabbaubar ist und mit einer dritten hämokompatiblen Schicht überzogen worden ist. Hierbei verleiht die äußere Schicht dem Stent die notwendige Blutverträglichkeit und die zweite Schicht dient als Wirkstoffreservoir. Der Wirkstoff, je nach Bedarf über eine hydrolyselabile Bindung kovalent an die Matrix gebunden oder/und der, für das Beschichtungsverfahren notwendig, in Lösungsmittel gelösten Matrix beigefügt, wird daher kontinuierlich und in geringen Konzentrationen aus der zweiten Schicht freigesetzt und diffundiert ungehindert durch die äußere hämokompatible Schicht. Auch dieser Schichtaufbau liefert das Resultat, dass der Bewuchs der Stentoberfläche mit Zellen nicht unterbunden, jedoch auf ein ideales Maß reduziert wird. Die erste Schicht bietet hierbei eine Risikominimierung für eventuelle eintretende Beschädigungen der beschichteten Stentoberfläche bei der Implantation, z. B. durch Abschürfungen durch die vorhandende Plaque oder in den Vorbereitungen z.B. beim Crimpen. Eine zweite Sicherheits-Gewährleistung ergibt sich durch die Tatsache, dass auch ein biostabiles Polymer sich im Körper durch eine mehr oder weniger langen Zeitraum abbauen wird und die Stentoberfläche zumindest teilweise freilegt
Kombinationen vor allem mit biodegradierbaren Material, wie in den Beschichtungsprinzipien beschrieben, sind hier ebenfalls möglich.

Derartige Stents lassen sich herstellen, indem man einen herkömmlichen Stent bereitstellt, auf dessen Oberfläche man eine hämokompatible erste Schicht aufbringt, eine nicht-bioabbaubare Schicht aufträgt, welche mindestens einen Wirkstoff als auch Kombinationen mit anderen Wirkstoffen aus anderen Gruppen enthält, kovalent oder/und adhäsiv gebunden, und diese Schicht im wesentlichen vollständig mit einer weiteren hämokompatiblen Schicht überzieht.
Substanzen, welche für die biostabile Schicht in Frage kommen, sind alle in der Medizin verwendeten beständigen Materialien, Dazu zählen Polyacrylsäure und Polyacrylate wie Polymethylmethacrylat, Polybutylmethacrylat, Polyacrylamid, Polyacrylonitrile, Polyamide, Polyetheramide, Polyethylenamin, Polyimide, Polycarbonate, Polycarbourethane, Polyvinylketone, Polyvinylhalogenide, Polyvinylidenhalogenide, Polyvinylether, Polyvinylaromaten, Polyvinylester, Polyvinylpyrollidone, Polyoxymethylene, Polyethylen, Polypropylen, Polytetrafluorethylen, Polyurethane, Polyolefin-Elastomere, Polyisobutylene, EPDM-Gummis, Fluorosilicone, Carboxymethylchitosan, Polyethylenterephtalat, Polyvalerate, Carboxymethylcellulose, Cellulose, Rayon, Rayontriacetate, Cellulosenitrate, Celluloseacetate, Hydroxyethylcellulose, Cellulosebutyrate, Celluloseacetat-butyrate, Ethylvinylacetat-copolymere, Polysulfone, Epoxyharze, ABS-Harze, EPDM-Gummis, Silicone wie Polysiloxane, Polyvinylhalogene und Copolymere, Celluloseether. Cellutosetriacetate. Chitosan und Copolymere und/oder Mischungen derselben.
Bei Mehrschichtsystemen überdeckt die neu aufgebrachte Schicht die darunterliegende Schicht im wesentlichen vollständig.

Die erfindungsgemäßen Stents lösen sowohl das Problem der akuten Thrombose als auch das Problem der Neolntimahyperplasie nach einer Stentimplantation. Zudem eignen sich die erfindungsgemäßen Stents aufgrund ihrer Beschichtung, ob als Einzelschicht oder als Mehrschichtsystem, besonders gut zur kontinuierlichen Freisetzung eines oder mehrerer antiproliferativer, immunsuppressiver und/oder Wirkstoffe. Aufgrund dieser Fähigkeit der gezielten kontinuierlichen Wirkstofffreisetzung in erforderlicher Menge verhindern die erfindungsgemäß beschichteten Stents die Gefahr der Restenose fast vollständig.

### Beispiele

### Beispiel 1

Kovalente hämokompatible Beschichtung von Stents:
Nicht expandierte Stents aus medizinischem Edelstahl LVM 316 wurden im Ultraschallbad 15 Minuten mit Aceton und Ethanol entfettet und bei 100°C im Trockenschrank getrocknet. Danach wurden sie für 5 Minuten in eine 2%ige Lösung von 3-Aminopropyltriethoxysilan in einem Gemisch Ethanol/Wasser (50/50 : (vlv)) getaucht und anschließend für 5 Minuten bei 100°C getrocknet. Anschließend wurden die Stents über Nacht mit demineralisiertem Wasser gewaschen.

### Beispiel 2

3 mg desultatiertes und reacetyliertes Heparin wurden bei 4°C in 30 ml 0,1 M MES-Puffer (2-(N-Morpholino)ethansutfonsäure) pH 4,75 gelöst und mit 30 mg N-Cyclohexyl-N'-(2-morpholinoethyl)carbodiimid-methyl-p-toluolsulfonat versetzt. In dieser Lösung wurden 10 Stents für 15 Stunden bei 4°C gerührt. Anschließend wurde mit Wasser, 4M NaCl-Lösung und Wasser für je 2 Stunden gespült.

### Beispiel 3 :

Bestimmung des Glucosamingehaltes der beschichteten Stents mittels HPLC Hydrolyse : Die beschichteten Stents werden in kleine Hydrolyserohre gegeben und mit 3ml 3M HCl genau eine Minute bei Raumtemperatur stehengelassen. Die Metallproben werden entfernt und die Röhrchen nach dem Verschließen 16h im Trockenschrank bei 100°C inkubiert. Danach lässt man abkühlen, es wird dreimal zur Trockne eingedampft und in 1 ml entgastem und filtriertem Wasser aufgenommen und gegen einen ebenfalls hydrolysierten Standard in der HPLC vermessen:

| Stent | Fläche Probe | desulfat+reacet. Heparin [g/Probe] | Fläche [cm]² | desulfat+reacet. Heparin [g/cm²] | desulfat+reacet. Heparin [pmol/cm²] |
|---|---|---|---|---|---|
| 1 | 129.021 | 2,7064TE-07 | 0,74 | 3.65739E-07 | 41,92 |
| 2 | 125.615 | 2.63502E-07 | 0,74 | 3,56084E-07 | 40,82 |
| 3 | 98,244 | 1.93072E-07 | 0,74 | 2,60908E-07 | 29,91 |
| 4 | 105.455 | 2,07243E-07 | 0,74 | 2,80058E-07 | 32,10 |
| 5 | 119,061 | 2,33982E-07 | 0,74 | 3,16192E-07 | 36,24 |
| 6 | 129,202 | 2,539911E-07 | 0,74 | 3,43124E-07 | 39,33 |
| 7 | 125,766 | 2,53957E-07 | 0,74 | 3.43185E-07 | 39,34 |

### Beispiel 4

Versuche zur Beschichtung von Oberflächen mit Tacrolimus:
Vorversuche mit Toluidinblau:
   Da Tacrolimus nur schlecht chemisch nachweisbar ist, werden erste Vorversuche mit Toluidinblau (Aldrich) durchgeführt.

### Chemikalien:

| | |
|---|---|
| Edelstahlröhrchen LVM 316 | : 2,5 cm lang, 2 mm Durchmesser |
| Polylaktid | : Fluka, Lot. 398555/123500, HNr. 0409 |
| Toluidinblau | : Aldrich, Lot. 19,816-1, HNr. 0430 |
| PBS-Puffer pH 7,4 | : 14,24 g Na₂HPO4, 2,72 g NaH₂PO₄ und 9 g NaCl |

### Durchführung:

Der Stent wird auf der Analysenwaage abgewogen und das Gewicht notiert. In einem kleinen Hydrolyserohr werden 0,5 g Polylaktid in 2 ml CHCl₃ gelöst und im Wasserbad auf ca. 65°C erwärmt. Die Lösung wird dann im Tiefkühlfach abgekühlt. Dazu werden 200 µg Toluidinblau in 200 µl CHCl₃ gegeben, In diese Lösung wird der Stent getaucht. Nach einigen Minuten wird der Stent mit einer Pinzette aus der Lösung genommen und im Abzug solange an der Luft bewegt, bis das Lösungsmittel verdampft ist. Danach wird der Stent ein zweites mal eingetaucht. Der Stent wird nach dem Trocknen an der Luft noch für ca. 10 min an die Gefriertrocknung gehängt. Nach dem Trocknen wird der Stent erneut gewogen. Aus der Gewichtsdifferenz errechnet sich die Menge des immobilisierten Polylaktids mit Toluidinblau. (Probe 1).
Dieser Versuch wird mit derselben Lösung ein weiteres Mal wiederholt (Probe 2).
Für Probe 3 wird die aus Versuch 1 (Probe 1) und Versuch 2 (Probe 2) resultierende Tauchlösung (1,93 ml) mit 0,825 mg Toluidinblau in 0,825 ml CHCl₃ und 250 mg Polylaktid versetzt. Das Polylaktid wird unter Erwärmung gelöst. Dann wird ein Stent zweimal darin wie oben beschrieben getaucht.

### Ergebnisse:

Die unbehandelten Stents hatten ein Gewicht von 176,0 mg und 180,9 mg. Nach dem Tauchen in die Polylaktidlösung wogen die Stents 200,9 und 205,2 mg.

Die Tauchlösung enthält 500 mg Polylaktid und 200 µg Toluidinblau aus diesem Verhältnis läßt sich die gebundene Toluidinblaumenge für die Proben 1 und 2 berechnen. Bei Probe 3 enthalten 2,755 ml Lösung 1 mg Toluidinblau und 638,6 mg Polylaktid (Einwaage-Verbrauch Probe 1 + 2; ca: 50 mg). Hier werden zwei Stents in einen Ansatz gegeben, um höhere Absorptionen zu erhalten. Da die Tauchlösung sehr zähflüssig war und sich dadurch eine sehr dicke Beschichtung ergab, wurde sie von 2,625 ml mit Chloroform auf 4 ml verdünnt.

### Konzentrationen in der Tauchlösung:

| Probe | Volumen (ml) | c (Polylactid mglml) | c (Toluidinblau µg/ml; |
|---|---|---|---|
| 1 | 2,2 | 227,3 | 90,9 |
| 2 | 2,2 | 227,3 | 90,9 |
| 3 | 2,755 | 231,8 | 363,0 |
| 4 | 4,0 | 134,5 | 212,5 |

### Gewicht der Röhrchen und die daraus errechnete Beschichtung :

| Probe | Leergewicht | Gesamtgewicht | PL u. Toluidinblau | Toluidinblau |
|---|---|---|---|---|
| 1 | 176,0 mg | 200,9 mg | 24,9 mg | 9,96 µg |
| 2 | 180,9 mg | 205,2 mg | 24,3 mg | 9,72 µg |
| 3 | 317,2 mg | 410,8 mg | 93,6 mg | 135,73 µg |
| 4 | 180,8 mg | 194,8 mg | 14,8 mg | 23,38 µg |

### Beispiel 5

Elutionsverhalten der Beschichtungen mit unterschiedlichen Konzentrationen:
Als Vorversuch wird ein UV-Vis Spektrum einer Toluidinblaulösung in Ethanol aufgenommen (C = 0,1 mg/ml) und das Absorptionsmaximum bestimmt. Die Toluidinblaukonzentration in der Lösung wird beim Absorptionsmaximum von 627 nm bestimmt. Dazu wird vorher eine Eichkurve erstellt.
Ein Stent wird in ein Becherglas mit 25 ml physiologischer Kochsalzlösung in einem Phosphatpuffer pH 7,4 (14,24 g NaH₂PO₄, 2,72 g K₂HPO₄ und 9 g NaCl) gehängt und vorsichtig bei Raumtemperatur gerührt. Nach 0,5, 1, 2, 3, 6, 24, 48 und 120 Stunden wird jeweils eine Probe von 3 ml entnommen, spektroskopisch vermessen und in den Ansatz zurückgegeben.

| Zeit/h | Abs. P1 | c(ng/ml) | Abs- P2 | c(ng/ml) | Abs. P3 | c (ng/ml) | Abs. P4 | c (ng/ml) |
|---|---|---|---|---|---|---|---|---|
| 0 | 0,0002 | 0 | -0,0002 | 0 | 0,0036 | 0 | 0,0063 | 0 |
| 0,5 | -0.0011 | 0 | 0,0011 | 6.4 | 0,0095 | 29,2 | 0,0125 | 30,7 |
| 1 | 0,0003 | 0,5 | 0,0014 | 7,9 | 0,0164 | 63,3 | 0,0121 | 28,7 |
| 2 | 0,0007 | 2,5 | 0,0006 | 5,0 | 0,0256 | 108.9 | 0,0131 | 33,7 |
| 3 | -0,0004 | 0 | 0.0006 | 4,0 | 0,0294 | 127,7 | 0,0136 | 36,1 |
| 6 | 0,0013 | 5.4 | 0.0015 | 8,4 | 0,0333 | 147,0 | 0,0142 | 39,1 |
| 24 | 0,0017 | 7.4 | 0,0020 | 10,8 | 0,0527 | 246,0 | 0,0239 | 176 |
| 48/96 | 0.0024 | 10,9 | 0.0033 | 17,3 | 0,1096 | 524,8 | 0,0147 | 41,6 |
| 120 | 0,0017 | 7,4 | 0,0038 | 19,8 | 0,1110 | 531,7 | 0,0161 | 48,5 |

Absorption der Proben nach unterschiedlichen Zeiten. Zur Berechnung der Konzentration wird die Küvettendifferenz (Abs. bei T =0) vom Meßwert abgezogen.

Nach 12 bzw. 13 Tagen wurde der Versuch abgebrochen. Auf allen Stents war nach Ablauf des Versuches noch eine Beschichtung vorhanden. Zur Bestimmung der in Lösung gegangenen Toluidinblau- bzw. Polylaktidmengen wurden die Stents mit Wasser und Ethanol gespült und danach für 1 h an die Gefriertrocknung gehängt, um sie dann zu wiegen.

| P. | Endgew. | Anfangsgew. | PL + Tb | gel. PL + Tb | gelöst Tolb. | Rest. Tolb. |
|---|---|---|---|---|---|---|
| 1 | 196,5 | 200,9 mg | 24,9 mg | 4,4 mg | 1,76 µg | 8,2 µg |
| 2 | 199,4 | 205,2 | 24,3 mg | 5,8 mg | 2,32 µg | 3,48 µg |
| 3 | 385,4 | 410,8 | 93,6 mg | 25,4 mg | 36,83 µg | 98,8 µg |
| 4 | 191,3 | 194,8 | 14,8 mg | 3,5 mg | 5,52 µg | 17,86 µg |

Bei Konzentrationen von 90 µg Toluidinblau pro ml Tauchlösung sind die freigesetzten Mengen Toluidinblau so gering, daß die Absorptionen an der Meßgrenze des Spektrometers liegen. Bei einer Konzentration von 200 µg/ml liegen die Werte nach wenigen Stunden im meßbaren Bereich. Es empfiehlt sich für die Messung zwei Proben in ein Becherglas (Elutionsgefäß) zu geben, um höhere Absorptionen zu erhalten. Bei der höchsten Polytaktid/Toluidinblaukonzentration scheint sich ein Sättigungseffekt einzustellen. Während die Elutionsrate bei den dünneren Proben nahezu linear verläuft. Auf allen Stents ist nach mehreren Tagen die Beschichtung noch immer zu erkennen.

Nach ca. 2 Wochen war im Mittel etwa 1/4 - 1/5 des gebunden Toluidinblau in Lösung gegangen. Daraus folgt,- daß die Proben noch ca. weitere 8 bis 10 Wochen Toluidinblau eluiert hätten.

Die Tauchlösung darf nicht zu dick sein und sollte gekühlt werden, damit das Chloroform beim Herausziehen der Proben nicht zu schnell verdampft da sonst die Dicke der Beschichtung zu groß und zu ungleichmäßig wird. Hier scheint die Polylaktidkonzentration in Probe 4 ausreichend (134 mg/ml). Zumal bei höheren Konzentrationen die Lösung extrem viskos wird und sich das Polylaktid nur sehr schwer lösen läßt.

### Beispiel 6

Beschichtung der Stents nach dem Sprühverfahren
Die nach Beispiel 1 und Beispiel 2 vorbereiteten nicht expandierten Stents werden gewogen und horizontal auf eine dünne Metallstange (d= 0,2mm) gehängt, die auf die Rotationsachse der Rotations- und Vorschubanlage gesteckt ist und mit 28 U/min rotiert. Die Stents werden so aufgebracht, das die Innenseite des Stents die Stange nicht berührt. Bei einer Vorschubamplitude von 2,2,cm und einer Vorschubgeschwindigkeit von 4cm/s und einem Abstand von 6 cm zwischen Stent und Düse wird der Stent mit der jeweiligen Sprühlösung besprüht. Nach dem Trocknen (ca 15 min) bei Raumtemperatur und folgend im Abzug über Nacht wird erneut gewogen.

### Beispiel 7

Beschichtung der Stents mit reiner Matrix
Herstellung der Sprühlösung :
176 mg Polylaktid wird eingewogen und mit Chloroform auf 20 g aufgefüllt.
Die Stents werden mit je 3 ml der Sprühlösung besprüht vor und nach dem Sprühen gewogen und die sich ergebende Schichtdicke durch Vermessung unter dem Mikroskop bei 100facher Vergrößerung ermittelt.

| Stent Nr. | Vor Beschichtung | Nach Beschichtung | Masse Beschichtung | Schichtdicke |
|---|---|---|---|---|
| 1 | 0,0193 g | 0,0205 g | 1,2 mg | 10,4 µm |
| 2 | 0,0193 g | 0,0205 g | 1,2 mg | 10,4 µm |
| 3 | 0,0204 g | 0,0216 g | 1,2 mg | 10,4 µm |
| 4 | 0,0206 g | 0,0217 g | 1,1 mg | 10,4 µm |

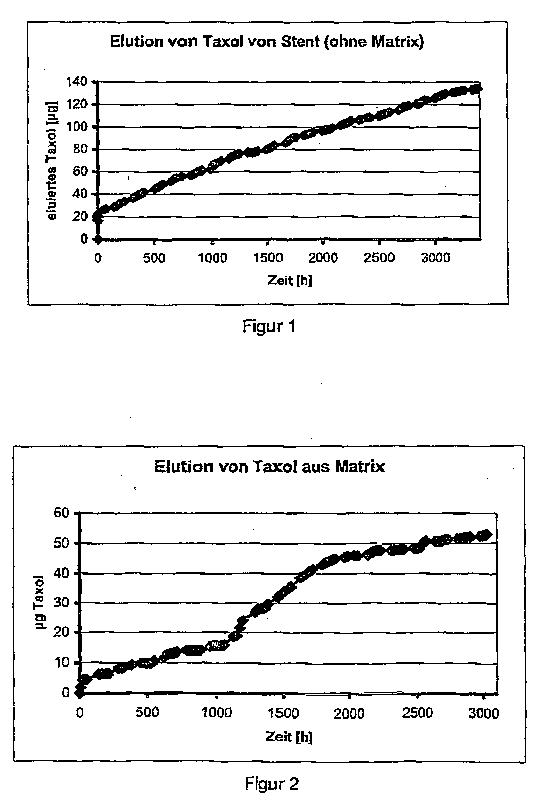

### Beispiel 8 (Figur 1)

Beschichtung der Stents mit reinem Wirkstoff ,
Herstellung der Sprühlösung : 44 mg Taxol werden in 6g Chloroform gelöst.
Die Stents werden vor und nach dem Sprühen gewogen.

| Stent Nr. | Vor Beschichtung | Nach Beschichtung | Masse Beschichtung |
|---|---|---|---|
| 1 | 0,0194 g | 0,0197 g | 0,30 mg |

### Beispiel 9

Ermittlung des Elutionsverhaltens in PBS-Puffer
Je ein Stent wird in einem genügend kleinen Gefäß mit 2 ml PBS-Puffer versetzt, mit Parafilm verschlossen und im Trockenschrank bei 37°C inkubiert. Nach Ablauf der gewählten Zeitintervalle wird jeweils der Überstand abpipettiert und dessen W-Absorption bei 306 nm gemessen.

### Beispiel 10

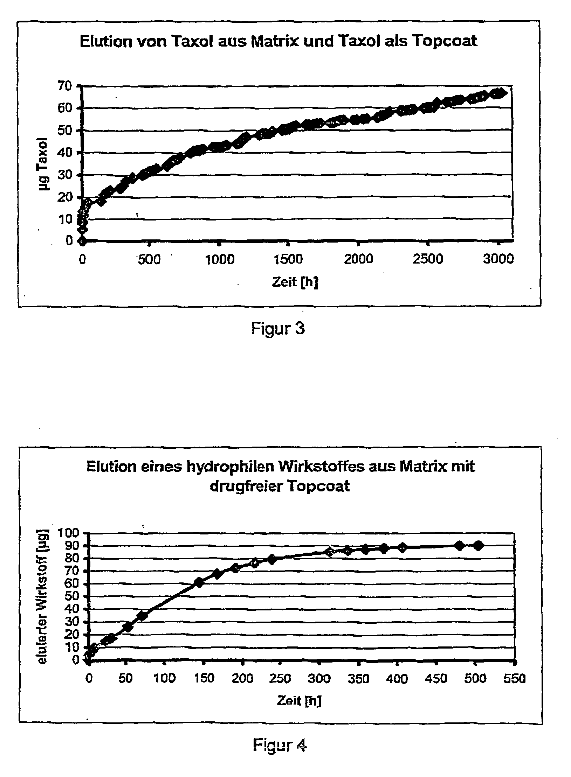

Beschichtung der hämokompatibel ausgerüsteten Stents mit wirkstoffbeladener Matrix (Figur 4)
Sprühlösung : Polylaktid RG502/Taxol - Lösung wird aus 145,2 mg Polylaktid und 48,4 mg Taxol auf 22 g mit Chloroform aufgefüllt.

| Stent | Sprühlösung | Gewicht vorh (g) | Gewicht nachher(g) | Masse Beschich | Masse Wirkstoff | Wirkstoff µg/mm² | Schicht dicke |
|---|---|---|---|---|---|---|---|
| 1 | 0,8 ml | 0,02180 | 0,02215 | 0,35 mg | 14.6 µg | 1,97 | 7,9 µm |
| 2 | 0,8 ml | 0,02105 | 0,02142 | 0,37 mg | 164 µg | 2,08 | 6,7 µm |
| 3 | 0,8 ml | 0,02247 | 0,02285 | 0,38 mg | 158 µg | 2,14 | 9,8 µm |
| 4 | 0,8 ml | 0,02395 | 0,02432 | 0,37 mg | 154 µg | 2,08 | 11,0 µm |
| 5 | 0,8 ml | 0,02247 | 0,02286 | 0,39 mg | 163 µg | 2,20 | 9,1 µm |
| 6 | 0,8 ml | 0,02442 | 0,02482 | 0,40 mg | 167 µg | 2,26 | 12,2 µm |

### Beispiel 11

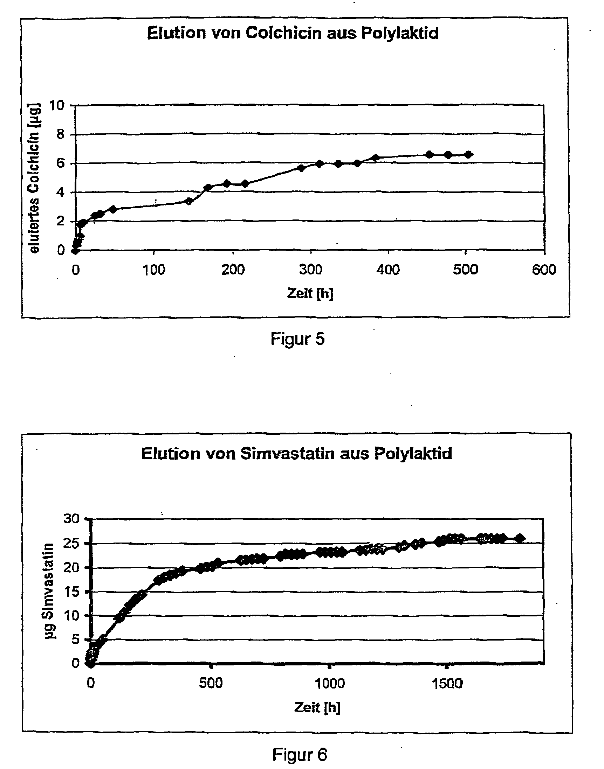

Beschichtung der Stents mit wirkstoffbeladener Matrix und Wirkstoff als Topcoat (Figur 5)
Basiscoat: 19,8 mg Polylakfid und 6,6 mg Taxol werden mit Chloroform auf 3 g aufgefüllt
Topcoat: 8,8 mg Taxol werden mit Chloroform auf 2 g aufgefüllt.

| Stent | Sprüh-Lösung | Gewicht vorh (g) | Gewicht nachher(g) | Masse Beschich | Masse Wirkstoff | Wirkstoff µg/mm² | Schicht dicke |
|---|---|---|---|---|---|---|---|
| 1 | 0,85 ml | 0,0235 | 0,0238 | 0,30 mg | 131 µg | 1,56 | 9,7µm |
| 2 | 0,85 ml | 0,0260 | 0,0264 | 0,40 mg | 175 µg | 2,09 | 10,1 µm |

### Beispiel 12

Beschichtung der Stents mit einen hydrophilen Wirkstoff enthaltenen Polylactid und einer wirkstofffreier Matrix als Topcoat (Figur 6)
Sprühlösungen
Basiscoat : 22 mg Polylaktid und 22 mg hydrophiler Wirkstoff werden eingewogen und mit Chloroform auf 5 g aufgefüllt.
Topcoat: 22 mg Polylaktid und 22 mg Polystyol werden eingewogen und mit Chloroform auf 5 g aufgefüllt.

| Sprühlösung | Vor Beschichtung | Nach Beschichtung | Masse Beschichtung | Masse Wirkstoff |
|---|---|---|---|---|
| 0,85 ml | 0,0135 g | 0,0143 g | 0,8 mg | 200 µg |

### Beispiel 13

Hämokompatibilität der verwendeten Matrix
4 Coronarstents : 2 unbehandelt, 2 beschichtet, nicht sterilisiert
Kennzeichnung : K3, K4 sind beschichtet
K5, K6 sind unbehandelt
Es werden folgende Messparameter bestimmt:
Blutbild
Plättchenfaktor 4 (PF4)
Complementfaktor 5a (C5a)
Thrombin-Antithrombin (TAT)

### Versuchsdurchführung :

Spenderblut wird in 1,5 U/ml Heparin aufgenommen. Die Stents werden in PVC-Schläuche (I.D. 3,5 mm, L=95cm) eingeführt und mittels Ballonkatheter fixiert. Die 4 Schläuche (K3-K6) und zwei Leerschläuche (L1, L2) werden mit jeweils 7,5 ml isoton. Kochsalzlösung gefülllt und 15 Minuten mit 5 U/min bei 37°C im Chandler-Loop rotiert. Die vollständig entleerten Schläuche werden mit heparinisiertem Spenderblut (7,5 ml) vorsichtig befüllt und 60 Min. bei 5 U/min rotiert. Entsprechend den Antikoagulantien werden Proben in Monovetten bzw. Probengefäßen entnommen (PF4 -CTAD, TAT-Citrat, C5a-EDTA, BB-EDTA) und weiterverarbeitet.

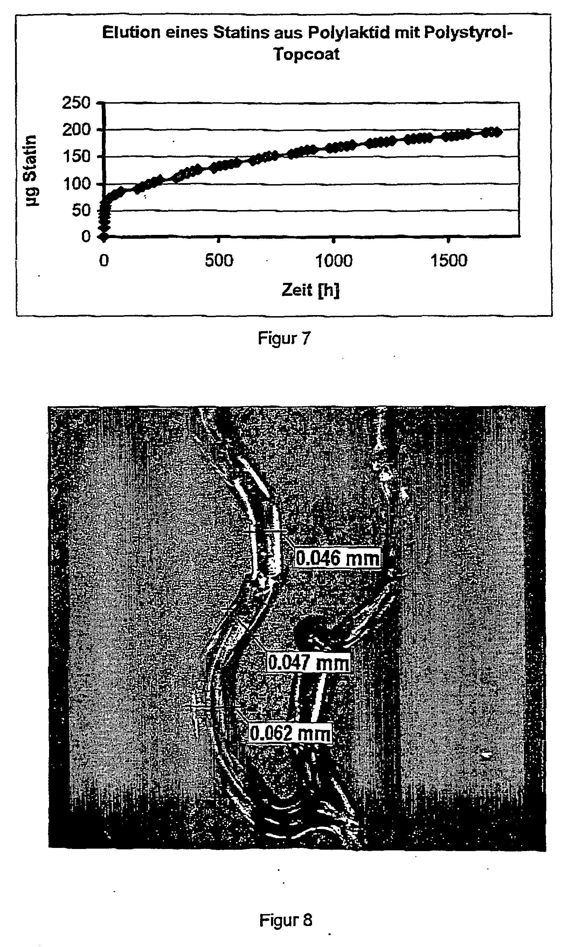

### Ergebnisse (Siehe Figur 8-10)

Die Bestimmung der Thrombocytenzahl zeigt keinen signifikanten Unterschied zwischen den leeren Kontrollschläuchen, den beschichteten und unbeschichteten Stents. Der freigesetzte PF4 liegt bei beschichteten und unbeschichteten Schläuchen auf gleichem Niveau, Die Bestimmung des aktivierten Komplementfaktors 5 (C5a) zeigt bei den beschichteten Stents eine geringere Aktivierung als bei den unbeschichteten Stents. Die Messung der TAT-Werte steht aus organisatorischen Gründen noch aus, Diese Proben werden bei -80°C gelagert.

### Beispiel 14

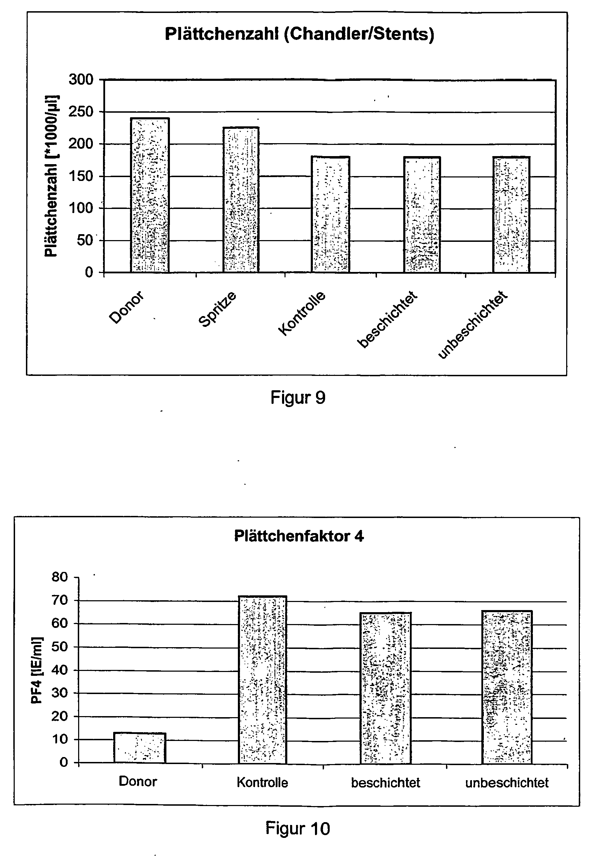

Ermittlung der Restenoserate im Tierversuch (Figur 10)
Jungschweine im Alter von 6-8 Monaten wurden je mit 4 Stents ausgestattet.
Es wurde ein unbehandelter Stent mit einem Polyacrylsäure beschichteten Stent und mit 2 kovalent an die Stentoberfläche gebundene hämokompatible Substanzen verglichen. Bei der einen Substanz handelt es sich um ein semisynthetisches Heparinderlvat, die andere Substanz ist die der Erythrocytenoberfläche entnommenen Oligo- und Polysaccharide der Glycocalix. Nach 4 Wochen werden die Tiere euthanasiert und die Restenosis-Rate bestimmt.

### Figurenbeschreibung

- **Figur 1:**: Elutionsdiagramm von Paclitaxel vom Stent (ohne Trägermaterial)
- **Figur 2 :**: Elutionsdiagramm von Paclitaxel eingebettet in Matrix
- **Figur 3 :**: Elutionsdiagramm von Paclitaxel eingebettet in Matrix und einer die Basisbeschichtung vollständig überziehender Schicht aus reinem Paclitaxel
- **Figur 4 :**: Elutionsdiagramm eines hydrophilen Wirkstoffes, eingelagert in der Matrix, und einem darüberliegenden, die Basisbeschichtung vollständig überziehendem Polymer (Topcoat) zur Diffusionskontrolle
- **Figur 5 :**: Elutionsdiagramm von Colchicin aus Matrix
- **Figur 6 :**: Elutionsdiagramm von Simvastatin aus Matrix
- **Figur 7 :**: Elutionsdiagramm eines Statins aus der Matrix mit Polystyrol als die Basisbeschichtung vollständig überziehende Diffusionskontrolle
- **Figur 8 :**: Aufnahme eines polymerbeschichteten Stents. Zur Kenntlichmachung der Beschichtung ist diese an einer Stelle aufgekratzt und es ist darunter deutlich die Oberfläche des Struts zu sehen.
- **Figur 8 :**: Vergleich der Thrombocytenzahl im Blut nach Chandler-Loop Zwischen beschichtetem (coated) und unbeschichtetem Stent (non coated) in Bezug zum Leerschlauch (Control), zur Plättchenzahl frisch entnommenen Blutes (Donor) und nach Lagerung von 60 Min in der Spritze (Syringe 60')
- **Figur 10 :**: Vergleich des Plättchenfaktor 4-Gehaltes im frisch entnommenen Blut (Donor), im Leerschlauch (Control) nach 60 Minuten und unbeschichteten Stents (non coated) mit beschichtetem Stent (coated)

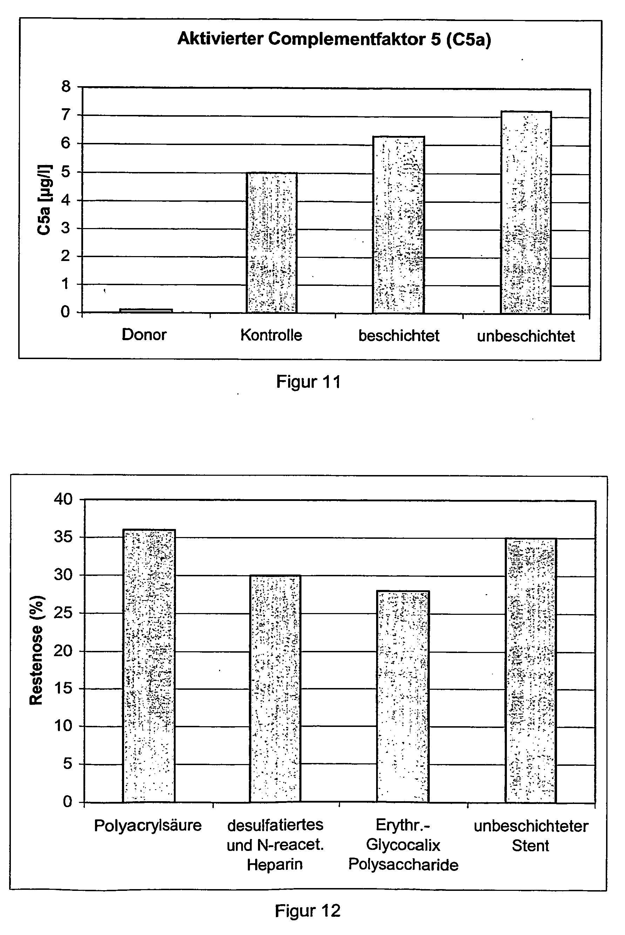

- **Figur 11:**: Vergleichendes Diagramm zum aktivierter Complementfaktor C5a im frisch entnommenen Blut (Donor), im Leerschlauch (Control) nach 60 Minuten und unbeschichteten Stents (non coated) mit beschichtetem Stent (coated)
- **Figur 12:**: Bildliche Darstellung der Restenose-Rate von mit vollständig desulfatiertem und N-reacetyliertem Heparin kovalent beschichteten Stents und mit Oligo- und Polysacchariden der Erythrocytenglycocalix beschichteten Stents im Vergleich zum unbeschichteten Stent und mit Polyacrylsäure beschichteten Stents (nach 4 Wochen Implantationszeit im Schwein)

## Patentansprüche

1. Stent, der mit einer hämokompatiblen Schicht überzogen ist und mit mindestens einer zweiten, darüberliegenden Schicht, welche mindestens einen antiproliferativen, antiinflammatorischen und/oder antithrombotischen Wirkstoff kovalent und/oder adhäsiv gebunden enthält, **dadurch gekennzeichnet, dass** die hämokompatible Schicht aus regioselektiv hergestellten Heparin-Derivaten unterschiedlicher Sulfatierungsgrade und Acetylierungsgrade im Molekulargewichtsbereich von dem für die antithrombotische Wirkung verantwortlichen Pentasaccharid bis zum Standardmolekulargewicht des käuflichen Heparins von ca. 13kD, aus Oligo- und Polysacchariden der Erythrocytenglycolcalix, aus desulfatiertem und N-reacetyliertem Heparin, aus N-carboxymethyliertem oder partiell N-acetyliertem Chitosan oder aus Mischungen dieser Substanzen besteht.

2. Stent gemäß Anspruch 1, **dadurch gekennzeichnet, daß** die Wirkstoffe aus der Gruppe ausgewählt werden, welche
Sirolimus, Rapamycin, Everolimus, Somatostatin, Tacrolimus, Roxithromycin, Dunaimycin, Ascomycin, Bafilomycin, Erythromycin, Midecamycin, Josamycin, Concanamycin, Clarithromycin, Troleandomycin, Folimycin, Cerivastatin, Simvastatin, Lovastatin, Fluvastatin, Rosuvastatin, Atorvastatin, Pravastatin, Pitavastatin, Vinblastin, Vincristin, Vindesin, Vinorelbin, Etobosid, Teniposid, Nimustin, Carmustin, Lomustin, Cyclophosphamid, 4-Hydroxyoxycyclophosphamid, Estramustin, Melphalan, Ifosfamid, Tropfosfamid, Chlorambucil, Bendamustin, Dacarbazin, Busulfan, Procarbazin, Treosulfan, Tremozolomid, Thiotepa, Daunorubicin, Doxorubicin, Aclarubicin, Epirubicin, Mitoxantron, Idarubicin, Bleomycin, Mitomycin, Dactinomycin, Methotrexat, Fludarabin, Fludarabin-5'-dihydrogenphosphat, Cladribin, Mercaptopurin, Thioguanin, Cytarabin, Fluorouracil, Gemcitabin, Capecitabin, Docetaxel, Carboplatin, Cisplatin, Oxaliplatin, Amsacrin, Irinotecan, Topotecan, Hydroxycarbamid, Miltefosin, Pentostatin, Aldesleukin, Tretinoin, Asparaginase, Pegasparase, Anastrozol, Exemestan, Letrozol, Formestan, Aminoglutethemid, Adriamycin, Azithromycin, Spiramycin, Cepharantin, SMC-Proliferation-Inhibitor-2w, Epothilone A und B, Mitoxanthrone, Azathioprin, Mycophenolatmofetil, c-myc-Antisense, b-myc-Antisense, Betulinsäure, Camptothecin, Lapachol, β-Lapachon,Podophyllotoxin, Betulin, Podophyllsäure-2-ethylhydrazid, Molgramostim, Peginterferon α-2b, Lanograstim, Filgrastim, Macrogol, Dacarbazin, Basiliximab, Daclizumab, Selectin, CETP-Inhibitor, Cadherine, Cytokininhibitoren, COX-2-Inhibitor, NFkB, Angiopeptin, Ciprofloxacin, Camptothecin, Fluroblastin, monoklonale Antikörper, die die Muskelzellproliferation hemmen, bFGF-Antagonisten, Probucol, Prostaglandine, 1,11-Dimethoxycanthin-6-on, 1-Hydroxy-11-Methoxycanthin-6-on, Scopolectin, Colchicin, NO-Donoren, Pentaerythrityltetranitrat, Syndnoeimine, S-Nitrosoderivate, Tamoxifen, Staurosporin, β-Estradiol, α-Estradiol, Estriol, Estron, Ethinylestradiol, Fosfestrol, Medroxyprogesteron, Estradiolcypionate, Estradiolbenzoate, Tranilast, Kamebakaurin und andere Terpenoide, die in der Krebstherapie eingesetzt werden, Verapamil, Tyrosin-Kinase-Inhibitoren, Tyrphostine, Cyclosporin A, Paclitaxel, 6-α-Hydroxy-Paclitaxel, Baccatin, Taxotere, synthetisch hergestellte als auch aus nativen Quellen gewonnene macrocyclische Oligomere des Kohlensuboxids (MCS) und seine Derivate, Mofebutazon, Acemetacin, Diclofenac, Lonazolac, Dapson, o-Carbamoylphenoxyessigsäure, Lidocain, Ketoprofen, Mefenaminsäure, Piroxicam, Meloxicam, Chloroquinphosphat, Penicillamin, Hydroxychloroquin, Auranofin, Natriumaurothiomalat, Oxaceprol, Celecoxib, β-Sitosterin, Ademetionin, Myrtecain, Polidocanol, Nonivamid, Levomenthol, Benzocain, Aescin, Ellipticin, D-24851 (Calbiochem), Colcemid, Cytochalasin A-E, Indanocine, Nocadazole, S 100 Protein, Bacitracin, Vitronectin-Rezeptor Antagonisten, Azelastin, Guanidylcyclase-Stimulator, Gewebsinhibitor der Metallproteinase-1 und 2, freie Nukleinsäuren, Nukleinsäuren in Virenüberträger inkorporiert, DNA- und RNA-Fragmente, Plaminogen-Aktivator Inhibitor-1, Plasminogen-Aktivator Inhibitor-2, Antisense Oligonucleotide, VEGF-Inhibitoren, IGF-1, Wirkstoffe aus der Gruppe der Antibiotika wie Cefadroxil, Cefazolin, Cefaclor, Cefotixin Tobramycin, Gentamycin, Penicilline, Dicloxacillin, Oxacillin, Sulfonamide, Metronidazol, Antithrombotika, Argatroban, Aspirin, Abciximab, synthetisches Antithrombin, Bivalirudin, Coumadin, Enoxoparin, desulfatiertens und N-reacetyliertes Heparin, Gewebe-Plasminogen-Aktivator, GpIIb/IIIa-Plättchenmembranrezeptor, Faktor Xₐ-Inhibitor Antikörper, Heparin, Hirudin, r-Hirudin, PPACK, Protamin, Prourokinase, Streptokinase, Warfarin, Urokinase, Vasodilatoren, Dipyramidol, Trapidil, Nitroprusside, PDGF-Antagonisten, Triazolopyrimidin und Seramin, ACE-Inhibitoren, Captopril, Cilazapril, Lisinopril, Enalapril, Losartan, Thioproteaseinhibitoren, Prostacyclin, Vapiprost, Interferon α, β und γ, Histaminantagonisten, Serotoninblocker, Apoptoseinhibitoren, Apoptoseregulatoren, p65 NF-kB, Bcl-xL-Antisense-Oligonukleotiden, Halofuginon, Nifedipin, Tocopherol Tranirast, Molsidomin, Teepolyphenole, Epicatechingallat, Epigallocatechingallat, Boswellinsäuren und ihre Derivate, Leflunomid, Anakinra, Etanercept, Sulfasalazin, Etoposid, Dicloxacyllin, Tetracyclin, Triamcinolon, Mutamycin, Procainimid, Retinolsäure, Quinidin, Disopyrimid, Flecainid, Propafenon, Sotolol, Amidoron, natürliche und synthetisch hergestellte Steroide, Bryophyllin A, Inotodiol, Maquirosid A, Ghalakinosid, Mansonin, Streblosid, Hydrocortison, Betamethason, Dexamethason, nichtsteroidale Substanzen, Fenoporfen, Ibuprofen, Indomethacin, Naproxen, Phenylbutazon, antivirale Agentien Acyclovir, Ganciclovir, Zidovudin, Antimykotika, Clotrimazol, Flucytosin, Griseofulvin, Ketoconazol, Miconazol, Nystatin, Terbinafin, antiprozoale Agentien Chloroquin, Mefloquin, Quinin, des weiteren natürliche Terpenoide, Hippocaesculin, Barringtogenol-C21-angelat, 14-Dehydroagrostistachin, Agroskerin, Agrostistachin, 17-Hydroxyagrostistachin, Ovatodiolide, 4,7-Oxycycloanisomelsäure, Baccharinoide B1, B2, B3 und B7, Tubeimosid, Bruceanole A,B und C, Bruceantinoside C, Yadanzioside N, und P, Isodeoxyelephantopin, Tomenphantopin A und B, Coronarin A,B,C und D, Ursolsäure, Hyptatsäure A, Zeorin, Iso-Iridogermanal. Maytenfoliol, Effusantin A, Excisanin A und B, Longikaurin B, Sculponeatin C, Kamebaunin, Leukamenin A und B, 13,18-Dehydro-6-alpha-Senecioyloxychaparrin, Taxamairin A und B, Regenilol, Triptolid, Cymarin, Apocymarin, Aristolochsäure, Anopterin, Hydroxyanopterin, Anemonin, Protoanemonin, Berberin, Cheliburinchlorid, Cictoxin, Sinococulin, Bombrestatin A und B, Cudraisoflavon A, Curcumin, Dihydronitidin, Nitidinchlorid, 12-beta-Hydroxypregnadien 3,20-dion, Bilobol, Ginkgol, Ginkgolsäure, Helenalin, Indicin, Indicin-N-oxid, Lasiocarpin, Inotodiol, Glykosid 1a, Podophyllotoxin, Justicidin A und B, Larreatin, Malloterin, Mallotochromanol, Isobutyrylmallotochromanol, Maquirosid A, Marchantin A, Maytansin, Lycoridicin, Margetin, Pancratistatin, Liriodenin, Bispsrthenolidin, Oxoushinsunin, Aristolactam-All, Bisparthenolidin, Periplocosid A, Ghalakinosid, Ursolsäure, Deoxypsorospermin, Psycorubin, Ricin A, Sanguinarin, Manwuweizsäure, Methylsorbifolin, Sphatheliachromen, Stizophyllin, Mansonin, Streblosid, Akagerin, Dihydrousambaraensin, Hydroxyusambarin, Strychnopentamin, Strychnophyllin, Usambarin, Usambarensin, Berberin, Liriodenin, Oxoushinsunin, Daphnoretin, Lariciresinol, Methoxylariciresinol, Syringaresinol, Umbelliferon, Afromoson, Acetylvismion B, Desacetylvismion A, Vismion A und B enthält.

3. Stent gemäß den vorangehenden Ansprüchen, **dadurch gekennzeichnet, dass** der antiproliferative, antiinflammatorische und/oder antithrombotische Wirkstoff in einer pharmazeutisch aktiven Konzentration von 0,001-10 mg pro cm² Stentoberfläche enthalten ist.

4. Stent gemäß Anspruch 1, wobei die hämokompatible Schicht adhäsiv oder bevorzugt kovalent an die Stentoberfläche gebunden ist.

5. Stent gemäß den vorangehenden Ansprüche, **dadurch gekennzeichnet, dass** die Beschichtung aus zwei oder mehr Schichten besteht, wobei die erste Schicht direkt auf die Stentoberfläche aufgebracht wird.

6. Stent gemäß Anspruch 5, **dadurch gekennzeichnet, dass** die erste Schicht aus einer hämokompatiblen Schicht besteht, welche vollständig oder unvollständig durch eine bioabbaubare und/oder biostabile Schicht überzogen ist, welche mindestens einen Wirkstoff gemäß Anspruch 2 in kovalent und/oder adhäsiv gebundener Form enthält.

7. Stent gemäß Anspruch 5 oder 6, **dadurch gekennzeichnet, daß** die zweite Schicht aus einer mindestens einen Wirkstoff gemäß Anspruch 2, in kovalent und/oder adhäsiv gebundener Form, enthaltenden nichtbioabbaubaren Schicht besteht, welche im wesentlichen vollständig mit einer bevorzugt kovalent gebundenen hämokompatiblen Schicht überzogen ist.

8. Stent gemäß Anspruch 6 oder 7, **dadurch gekennzeichnet, dass** als bioabbaubare Substanzen für die bioabbaubare Schicht Polyvalerolactone, Poly-ε-Decalactone, Polylactonsäure, Polyglycolsäure Polylactide, Polyglycolide, Copolymere der Polylactide und Polyglycolide, Poly-ε-caprolacton, Polyhydroxybuttersäure, Polyhydroxybutyrate, Polyhydroxyvalerate, Polyhydroxybutyrate-co-valerate, Poly(1,4-dioxan-2,3-dione), Poly(1,3-dioxan-2-one), Poly-para-dioxanone, Polyanhydride, Polymaleinsäure-anhydride, Polyhydroxymethacrylate, Fibrin, Polycyanoacrylate, Polycaprolactondimethylacrylate, Poly-b-Maleinsäure Polycaprolactonbutyl-acrylate, Multiblockpolymere aus Oligocaprolactondiole und Oligodioxanondiole, Polyetherestermultiblockpolymere aus PEG und Poly(butylenterephtalat), Polypivotolactone, Polyglycolsäuretrimethyl-carbonate Polycaprolacton-glycolide, Poly(g-ethylglutamat), Poly(DTH-Iminocarbonat), Poly(DTE-co-DT-carbonat), Poly(Bisphenol A-iminocarbonat), Polyorthoester, Polyglycol-säuretrimethylcarbonate, Polytrimethylcarbonate Polyiminocarbonate, Poly(N-vinyl)-Pyrolidon, Polyvinylalkohole, Polyesteramide, glycolierte Polyester, Polyphosphoester, Polyphosphazene, Poly[p-carboxyphenoxy)propan] Polyhydroxypentansäure, Polyanhydride, Polyethylenoxid-propylenoxid, weiche Polyurethane, Polyurethane mit Aminosäurereste im Backbone, Polyetherester, Polyethylenoxid, Polyalkenoxalate, Polyorthoester sowie deren Copolymere, Lipide, Carrageenane, Fibrinogen, Stärke, Kollagen, protein-basierende Polymere, Polyaminosäuren, synthetische Polyaminosäuren, Zein, modifiziertes Zein, Polyhydroxyalkanoate, Pectinsäure, Actinsäure, modifiziertes und unmodifiziertes Fibrin und Casein, Carboxymethylsulfat, Albumin, Hyaluronsäure, Heparansulfat, Heparin, Chondroitinsulfat, Dextran, β-Cyclodextrine, Copolymere mit PEG und Polypropylenglycol, Gummi arabicum, Guar, Gelatine, Collagen Collagen-N-Hydroxysuccinimid, Lipide, Phospholipide, Modifikationen und Copolymere und/oder Mischungen der vorgenannten Substanzen eingesetzt werden.

9. Stent gemäß Anspruch 6, 7 oder 8, **dadurch gekennzeichnet, dass** als biostabile Substanzen für die biostabile Schicht Polyacrylsäure und Polyacrylate, Polymethylmethacrylat, Polybutylmethacrylat, Polyacrylamid, Polyacrylonitrile, Polyamide, Polyetheramide, Polyethylenamin, Polyimide, Polycarbonate, Polycarbourethane, Polyvinylketone, Polyvinylhalogenide, Polyvinylidenhalogenide, Polyvinylether, Polyisobutylene, Polyvinylaromaten, Polyvinylester, Polyvinylpyrollidone, Polyoxymethylene, Polytetramethylenoxid, Polyethylen, Polypropylen, Polytetrafluorethylen, Polyurethane, Polyetherurethane Silicon-Polyetherurethane, Silicon-Polyurethane, Silicon-Polycarbonat-Urethane, Polyolefin-Elastomere, Polyisobutylene, EPDM-Gummis, Fluorosilicone, Carboxymethylchitosan, Polyaryletheretherketone, Polyetheretherketone, Polyethylenterephtalat, Polyvalerate, Carboxymethylcellulose, Cellulose, Rayon, Rayontriacetate, Cellulosenitrate, Celluloseacetate, Hydroxyethylcellulose, Cellulosebutyrate, Celluloseacetatbutyrate, Ethylvinylacetat-copolymere, Polysulfone, Epoxyharze, ABS-Harze, EPDM-Gummis, Silicone, Polysiloxane, Polydimethylsiloxane, Polyvinylhalogene und Copolymere, Celluloseether, Cellulosetriacetate, Chitosan und Copolymere und/oder Mischungen derselben eingesetzt werden.

10. Verfahren zur hämokompatiblen, antiproliferativen, antiinflammatorischen und/oder athrombogenen Beschichtung von Stents gemäß Anspruch 1 **gekennzeichnet durch** die Schritte:
a) Bereitstellen eines unbeschichteten Stents;
b) Aufbringen einer bevorzugt kovalent gebundenen hämokompatiblen Schicht,
c) im wesentlichen vollständiges Überziehen der hämokompatiblen Schicht im Tauch- oder Sprühverfahren mit mindestens einem Wirkstoff und/oder **durch** kovalente Kopplung des Wirkstoffes an die darunterliegende Schicht, oder
c') im wesentlichen vollständiges Überziehen oder unvollständiges Überziehen der hämokompatiblen Schicht im Tauch- oder Sprühverfahren mit mindestens einer bioabbaubaren und/oder biostabilen Schicht, welche mindestens einen Wirkstoff kovalent und/oder adhäsiv gebunden enthält.

11. Verfahren nach Anspruch 10, **gekennzeichnet durch** die Schritte a), b), c') und
d) im wesentlichen vollständiges Überziehen der hämokompatiblen Schicht mit mindestens einer bioabbaubaren und/oder biostabilen Schicht, welche mindestens einen Wirkstoff kovalent und/oder adhäsiv gebunden enthält und eine weitere, die darunterliegende Schicht vollständig und/oder teilweise überdeckende bioabbaubare und/oder biostabile Schicht ohne Wirkstoff als Diffusionsbarriere.

12. Verfahren nach Anspruch 10, **gekennzeichnet durch** die Schritte a), b) und
c') Aufbringen einer biostabilen Schicht, welche mindestens einen antiproliferativen, antiinflammatorschen und/oder antithrombotischen Wirkstoff kovalent und/oder adhäsiv gebunden enthält; und
d) im wesentlichen vollständiges Überziehen der biostabilen Schicht mit einer hämokompatiblen Schicht.

13. Verfahren nach Anspruch 10, **gekennzeichnet durch** die Schritte a), b), c') und
d) Aufbringen eines Wirkstoffes oder einer Wirkstoffkombination, kovalent und/oder adhäsiv an die darunterliegende Schicht,
e) im wesentlichen vollständiges Überziehen der hämokompatiblen Schicht mit mindestens einer bioabbaubaren und/oder biostabilen Schicht, welche mindestens einen Wirkstoff kovalent und/oder adhäsiv gebunden enthält,
f) Aufbringen eines Wirkstoffes oder einer Wirkstoffkombination kovalent und/oder adhäsiv gebunden und eine weitere, die darunterliegende Schicht vollständig oder teilweise überdeckende bioabbaubare und/oder biostabile Schicht ohne Wirkstoff als Diffusionsbarriere.

14. Verfahren nach Anspruch 10, **gekennzeichnet durch** die Schritte a), b) und
c') im wesentlichen vollständiges oder unvollständiges Überziehen der hämokompatiblen Schicht mit mindestens zwei bioabbaubaren und/oder biostabilen Schichten, welche mindestens einen Wirkstoff kovalent und/oder adhäsiv gebunden in unterschiedlicher Konzentration je Schicht enthalten, oder
d) im wesentlichen vollständiges oder unvollständiges Überziehen der hämokompatiblen Schicht mit mindestens zwei bioabbaubaren oder/und biostabilen Schichten, welche mindestens einen Wirkstoff kovalent und/oder adhäsiv gebunden in unterschiedlicher Konzentration je Schicht enthalten und mindestens eine weitere, die darunterliegende Schicht vollständig oder teilweise überdeckende, bioabbaubare und/oder biostabile Schicht ohne Wirkstoff als Diffusionsbarriere, oder
e) im wesentlichen vollständiges oder unvollständiges Überziehen der hämokompatiblen Schicht mit mindestens einer bioabbaubaren und/oder biostabilen Schicht, welche mindestens einen Wirkstoff und mindestens einen weiteren Wirkstoff derselben Gruppe oder aus einer anderen Gruppe mit komplementären Eigenschaften in gleicher oder unterschiedlicher Konzentrationen kovalent und/oder adhäsiv gebunden enthält, oder
f) im wesentlichen vollständiges oder unvollständiges Überziehen der hämokompatiblen Schicht mit mindestens zwei bioabbaubaren und/oder biostabilen Schichten, welche mindestens einen Wirkstoff und mindestens einen weiteren Wirkstoff derselben Gruppe oder aus einer anderen Gruppe mit komplementären Eigenschaften in gleicher oder unterschiedlicher Konzentrationen kovalent und/oder adhäsiv gebunden enthalten und mindestens eine weitere, die darunterliegende Schicht vollständig und/oder teilweise überdeckende, bioabbaubare und/oder biostabile Schicht ohne Wirkstoff als Diffusionsbarriere, oder
g) im wesentlichen vollständiges Überziehen der hämokompatiblen Schicht mit mindestens zwei bioabbaubaren und/oder biostabilen Schichten, welche mindestens einen Wirkstoff in gleichen und/oder unterschiedlichen Konzentrationen kovalent und/oder adhäsiv gebunden enthalten und einer weiteren, die darunterliegende Schicht vollständig oder auch nur teilweise überdeckende, bioabbaubare und/oder biostabile Schicht ohne Wirkstoff als Diffusionsbarriere und einer diese Schicht überziehenden Wirkstoffschicht, bestehend aus mindestens einem Wirkstoff kovalent und/oder adhäsiv an die darunterliegende Schicht gebunden.

15. Verfahren nach einem der Ansprüche 10 - 14, **dadurch gekennzeichnet, daß** die hämokompatiblen Schichten aus Heparin nativen Ursprungs als auch regioselektiv hergestellter Derivate unterschiedlicher Sulfatierungsgrade und Acteylierungsgrade im Molekulargewichtsbereich von des für die antithrombotische Wirkung verantwortlichen Pentasaccharides bis zum Standardmolekulargewicht des käuflichen Heparins von ca. 13kD, Heparansulfate und seine Derivate, Oligo- und Polysaccharide der Erythrocytenglycolcalix, Oligosaccharide, Polysaccharide, vollständig desulfatiertes und N-reacetyliertes Heparin, desulfatiertes und N-reacetyliertes Heparin, N-carboxymethyliertes und/oder partiell N-acetyliertes Chitosan, Polyacrylsäure, Polyvinylpyrrolidon, und/oder Polyetheretherketon, Polyethylenglykol oder aus Mischungen dieser Substanzen bestehen.

16. Verfahren nach einem der Ansprüche 10 - 15, **dadurch gekennzeichnet, dass** die hämokompatible erste Schicht bevorzugt kovalent an den Stent gebunden ist, die biostabile Schicht die erste Schicht vollständig oder unvollständig überzieht und die dritte hämokompatible Schicht bevorzugt kovalent an die zweite Schicht gebunden ist.

## Claims

1. Stent, covered with a hemocompatible coating and at least a second overlying coating which contains at least one anti-proliferative, anti-inflammatory and/or anti-thrombotic active agent covalently and/or adhesively bound, **characterized in that** the hemocompatible coating consists of regioselectively produced heparin derivatives of different degrees of sulfation and acetylation with a molecular weight ranging from the pentasaccharide responsible for the anti-thrombotic effect to the standard molecular weight of commercially available heparin of ca. 13kD, of oligo- and polysaccharides of the glycocalix of erythrocytes, of desulfated and N-reacetylated heparin, of N-carboxymethylated or partially N-acetylated chitosan, or of compositions of these substances.

2. Stent according to claim 1, **characterized in that** the active agents are chosen from the group which contains sirolimus, rapamycin, everolimus, somatostatin, tacrolimus, roxithromycin, dunaimycin, ascomycin, bafilomycin, erythromycin, midecamycin, josamycin, concanamycin, clarithromycin, troleandomycin, folimycin, cerivastatin, simvastatin, lovastatin, fluvastatin, rosuvastatin, atorvastatin, pravastatin, pitavastatin, vinblastine, vincristine, vindesine, vinorelbine, etoboside, teniposide, nimustine, carmustine, lomustine, cyclophosphamide, 4-hydroxyoxycyclophosphamide, estramustine, melphalan, ifosfamide, trofosfamide, chlorambucil, bendamustine, dacarbazine, busulfan, procarbazine, treosulfan, tremozolomide, thiotepa, daunorubicin, doxorubicin, aclarubicin, epirubicin, mitoxantron, idarubicin, bleomycin, mitomycin, dactinomycin, methotrexate, fludarabine, fludarabine-5'-dihydrogenphosphate, cladribine, mercaptopurine, thioguanine, cytarabine, fluorouracil, gemcitabine, capecitabine, docetaxel, carboplatin, cisplatin, oxaliplatin, amsacrine, irinotecan, topotecan, hydroxycarbamide, miltefosine, pentostatin, aldesleukin, tretinoin, asparaginase, pegasparase, anastrozole, exemestane, letrozole, formestane, aminoglutethemide, adriamycin, azithromycin, spiramycin, cepharanthin, SMC proliferation inhibitor-2w, epothilone A and B, mitoxantrone, azathioprine, mycophenolate mofetil, c-myc antisense, b-myc antisense, betulinic acid, camptothecin, lapachol, β-lapachone, podophyllotoxin, betulin, podophyllic acid 2-ethylhydrazide, molgramostim, peginterferon α-2b, lenograstim, filgrastim, macrogol, dacarbazine, basiliximab, daclizumab, selectin, CETP inhibitor, cadherins, cytokinin inhibitors, COX-2 inhibitor, NFkB, angiopeptin, ciprofloxacin, camptothecin, fluroblastin, monoclonal antibodies, which inhibit the muscle cell proliferation, bFGF antagonists, probucol, prostaglandins, 1,11-dimethoxycanthin-6-one, 1-hydroxy-11-methoxycanthin-6-one, scopoletin, colchicine, NO donors, pentaerythrityl tetranitrate, sydnonimines, S-nitrosoderivatives, tamoxifen, staurosporine, β-estradiol, α-estradiol, estriol, estrone, ethinylestradiol, fosfestrol, medroxyprogesterone, estradiol cypionates, estradiol benzoates, tranilast, kamebakaurin and other terpenoids, which are applied in the therapy of cancer, verapamil, tyrosine kinase inhibitors, tyrphostine, cyclosporine A, paclitaxel, 6-α-hydroxy-paclitaxel, baccatin, taxotere, macrocyclic oligomers of carbon suboxide (MCS) obtained synthetically and from native sources and derivatives thereof, mofebutazone, acemetacin, diclofenac, lonazolac, dapsone, o-carbamoyl-phenoxy-acetic acid, lidocaine, ketoprofen, mefenamic acid, piroxicam, meloxicam, chloroquine phosphate, penicillamine, hydroxychloroquine, auranofin, sodium aurothiomalate, oxaceprol, celecoxib, β-sitosterin, ademetionine, myrtecaine, polidocanol, nonivamide, levomenthol, benzocaine, aescin, ellipticine, D-24851 (Calbiochem), colcemid, cytochalasin A-E, indanocine, nocadazole, S 100 protein, bacitracin, vitronectin receptor antagonists, azelastine, guanidyl cyclase stimulator, tissue inhibitor of metal proteinase-1 and -2, free nucleic acids, nucleic acids incorporated into virus transmitters, DNA and RNA fragments, plaminogen activator inhibitor-1, plasminogen activator inhibitor-2, antisense oligonucleotides, VEGF inhibitors, IGF-1, active agents from the group of antibiotics such as cefadroxil, cefazolin, cefaclor, cefoxitin, tobramycin, gentamycin, penicillin, dicloxacillin, oxacillin, sulfonamides, metronidazole, antithrombotics, argatroban, aspirin, abciximab, synthetic antithrombin, bivalirudin, coumadin, enoxaparin, desulfated and N-reacetylated heparin, tissue plasminogen activator, GpIIb/IIIa platelet membrane receptor, factor Xₐ inhibitor antibody, heparin, hirudin, r-hirudin, PPACK, protamine, prourokinase, streptokinase, warfarin, urokinase, vasodilators, dipyridamol, trapidil, nitroprussides, PDGF antagonists, triazolopyrimidine and seramin, ACE inhibitors, captopril, cilazapril, lisinopril, enalapril, losartan, thioprotease inhibitors, prostacyclin, vapiprost, interferon α, β and γ, histamine antagonists, serotonin blockers, apoptosis inhibitors, apoptosis regulators, p65 NF-kB, Bcl-xL antisense oligonucleotides, halofuginone, nifedipine, tocopherol, tranirast, molsidomine, tea polyphenols, epicatechin gallate, epigallocatechin gallate, Boswellic acids and derivatives thereof, leflunomide, anakinra, etanercept, sulfasalazine, etoposide, dicloxacillin, tetracycline, triamcinolone, mutamycin, procainimide, retinoic acid, quinidine, disopyrimide, flecainide, propafenone, sotolol, amidorone, natural and synthetically obtained steroids, bryophyllin A, inotodiol, maquiroside A, ghalakinoside, mansonine, strebloside, hydrocortisone, betamethasone, dexamethasone, non-steroidal substances, fenoprofen, ibuprofen, indomethacin, naproxen, phenylbutazone, antiviral agents, acyclovir, ganciclovir, zidovudine, antimycotics, clotrimazole, flucytosine, griseofulvin, ketoconazole, miconazole, nystatin, terbinafine, antiprotozoal agents, chloroquine, mefloquine, quinine, moreover natural terpenoids, hippocaesculin, barringtogenol-C21-angelate, 14-dehydroagrostistachin, agroskerin, agrostistachin, 17-hydroxyagrostistachin, ovatodiolids, 4,7-oxycycloanisomelic acid, baccharinoids B1, B2, B3 and B7, tubeimoside, bruceanol A, B and C, bruceantinoside C, yadanziosides N and P, isodeoxyelephantopin, tomenphantopin A and B, coronarin A, B, C and D, ursolic acid, hyptatic acid A, zeorin, iso-iridogermanal, maytenfoliol, effusantin A, excisanin A and B, longikaurin B, sculponeatin C, kamebaunin, leukamenin A and B, 13,18-dehydro-6-α-senecioyloxychaparrin, taxamairin A and B, regenilol, triptolide, cymarin, apocymarin, aristolochic acid, anopterin, hydroxyanopterin, anemonin, protoanemonin, berberine, cheliburin chloride, cicutoxin, sinococuline, bombrestatin A and B, cudraisoflavone A, curcumin, dihydronitidine, nitidine chloride, 12-β-hydroxypregnadien-3,20-dione, bilobol, ginkgol, ginkgolic acid, helenalin, indicine, indicine-N-oxide, lasiocarpine, inotodiol, glycoside 1a, podophyllotoxin, justicidin A and B, larreatin, malloterin, mallotochromanol, isobutyrylmallotochromanol, maquiroside A, marchantin A, maytansine, lycoridicin, margetine, pancratistatin, liriodenine, bisparthenolidine, oxoushinsunine, aristolactam-All, bisparthenolidine, periplocoside A, ghalakinoside, ursolic acid, deoxypsorospermin, psycorubin, ricin A, sanguinarine, manwu wheat acid, methylsorbifolin, sphatheliachromen, stizophyllin, mansonine, strebloside, akagerine, dihydrousambaraensine, hydroxyusambarine, strychnopentamine, strychnophylline, usambarine, usambarensine, berberine, liriodenine, oxoushinsunine, daphnoretin, lariciresinol, methoxylariciresinol, syringaresinol, umbelliferone, afromoson, acetylvismione B, desacetylvismione A, vismione A and B.

3. Stent according to the previous claims, **characterized in that** the anti-proliferative, anti-inflammatory and/or anti-thrombotic active agent is contained in a pharmaceutically active concentration of 0.001-10 mg / cm² of the stent surface.

4. Stent according to claim 1, wherein the hemocompatible coating is adhesively, or preferred, covalently bound to the stent surface.

5. Stent according to the previous claims, **characterized in that** the coating consists of two or more layers, wherein the first layer is applied directly to the stent surface.

6. Stent according to claim 5, **characterized in that** the first layer consists of a hemocompatible coating that is completely or incompletely covered with a biodegradable and/or biostable layer containing at least one active agent according to claim 2 bound in a covalent and/or adhesive form.

7. Stent according to claim 5 or 6, **characterized in that** the second layer consists of a non-biodegradable layer containing at least one active agent according to claim 2 bound in a covalent and/or adhesive form, wherein this layer is substantially completely covered with a preferably covalently bound hemocompatible coating.

8. Stent according to claims 6 or 7, **characterized in that** polyvalerolactones, poly-ε-decalactones, polylactonic acid, polyglycolic acid, polylactides, polyglycolides, copolymers of the polylactides and polyglycolides, poly-ε-caprolactone, polyhydroxybutanoic acid, polyhydroxybutyrates, polyhydroxyvalerates, polyhydroxybutyrate-co-valerates, poly(1,4-dioxane-2,3-diones), poly(1,3-dioxane-2-one), poly-para-dioxanones, polyanhydrides, polymaleic anhydrides, polyhydroxymethacrylates, fibrin, polycyanoacrylates, polycaprolactonedimethylacrylates, poly-b-maleic acid, polycaprolactonebutyl-acrylates, multiblock polymers from oligocaprolactonedioles and oligodioxanonedioles, polyetherester multiblock polymers from PEG and poly(butyleneterephtalates), polypivotolactones, polyglycolic acid trimethyl-carbonates, polycaprolactone-glycolides, poly(g-ethylglutamate), poly(DTH-iminocarbonate), poly(DTE-co-DT-carbonate), poly(bisphenol-A-iminocarbonate), polyorthoesters, polyglycolic acid trimethyl-carbonates, polytrimethylcarbonates, polyiminocarbonates, poly(N-vinyl)-pyrrolidone, polyvinylalcoholes, polyesteramides, glycolated polyesters, polyphosphoesters, polyphosphazenes, poly[p-carboxyphenoxy)propane], polyhydroxypentane acid, polyanhydrides, polyethyleneoxide-propyleneoxide, soft polyurethanes, polyurethanes with amino acid residues in the backbone, polyetheresters, polyethyleneoxide, polyalkeneoxalates, polyorthoesters as well as their copolymers, lipids, carrageenanes, fibrinogen, starch, collagen, protein based polymers, polyamino acids, synthetic polyamino acids, zein, modified zein, polyhydroxyalkanoates, pectic acid, actinic acid, modified and non-modified fibrin and casein, carboxymethylsulfate, albumin, hyaluronic acid, heparansulphates, heparin, chondroitinesulphate, dextran, β-cyctodextrines, copolymers with PEG and polypropyleneglycol, gummi arabicum, guar, gelatine, collagen, collagen-N-Hydroxysuccinimide, lipids, phospholipids, modifications and copolymers and/ or mixtures of these substances are used as biodegradable substances for the biodegradable layer.

9. Stent according to claims 6, 7 or 8 , **characterized in that** polyacrylic acid and polyacrylates, polymethylmethacrylate, polybutylmethacrylate, polyacrylamide, polyacrylonitriles, polyamides, polyetheramides, polyethylenamine, polyimides, polycarbonates, polycarbourethanes, polyvinyl ketones, polyvinyl halogenides, polyvinylidene halogenides, polyvinyl ethers, polyisobutylenes, polyvinyl aromates, polyvinyl esters, polyvinyl pyrrollidones, polyoxymethylenes, polytetramethylene oxide, polyethylene, polypropylene, polytetrafluoro-ethylene, polyurethanes, polyether urethanes, silicone-polyether-urethanes, silicone-polyurethanes, silicone-polycarbonate-urethanes, polyolefine elastomers, polyisobutylenes, EPDM gums, fluorosilicones, carboxymethylchitosans, polyaryletherether ketones, polyetherether ketones, polyethylene terephthalate, polyvalerates, carboxymethylcellulose, cellulose, rayon, rayon triacetates, cellulose nitrates, cellulose acetates, hydroxyethyl cellulose, cellulose butyrates, cellulose acetate butyrates, ethyl vinyl acetate copolymers, polysulphones, epoxy resins, ABS resins, EPDM gums, silicones, polysiloxanes, polydimethylsiloxanes, polyvinyl halogens and copolymers, cellulose ethers, cellulose triacetates, chitosan and copolymers and/ or mixtures of these substances are used as biostable substances for the biostable layer.

10. Method for the hemocompatible, anti-proliferative, anti-inflammatory and/or athrombogenic coating of stents according to claim 1, **characterized by** the steps:
a) Providing an uncoated stent
b) Applying a hemocompatible coating, preferably covalently bound
c) Substantially complete covering of the hemocompatible coating by dip coating or spray coating with at least one active agent and/or by covalent coupling of the active agent to the subjacent layer,
or
c') Substantially complete covering or incomplete covering of the hemocompatible coating by dip coating or spray coating with at least one biodegradable and/or biostable layer containing at least one active agent covalently and/or adhesively bound.

11. Method according to claim 10 , **characterized by** the steps a), b), c') and
d) Substantially complete covering of the hemocompatible coating with at least one biodegradable and/or biostable layer containing at least one active agent covalently and/or adhesively bound, and a further biodegradable and/or biostable layer as a diffusion barrier without an active agent, covering the subjacent layer completely and/or partially.

12. Method according to claim 10 , **characterized by** the steps a), b) and
c') Applying a biostable layer containing at least one anti-proliferative, anti-inflammatory and/or anti-thrombotic active agent covalently and/or adhesively bound; and
d) Substantially complete covering of the biostable layer with a hemocompatible coating.

13. Method according to claim 10, **characterized by** the steps a), b) and c') and
d) Applying an active agent or a combination of active agents, covalently and/or adhesively bound to the subjacent layer,
e) Substantially complete covering of the hemocompatible coating with at least one biodegradable and/or biostable layer containing at least one active agent covalently and/or adhesively bound,
f) Applying an active agent or a combination of active agents covalently and /or adhesively bound and a further biodegradable and/or biostable layer as a diffusion barrier without an active agent, covering the subjacent layer completely and/or partially.

14. Method according to claim 10, **characterized by** the steps a), b) and
c') Substantially complete or incomplete covering of the hemocompatible coating with at least two biodegradable and/or biostable layers containing at least one active agent covalently and/or adhesively bound in different concentration per layer, or
d) Substantially complete or incomplete covering of the hemocompatible coating with at least two biodegradable and/or biostable layers containing at least one active agent covalently and/or adhesively bound in different concentration per layer and at least one further biodegradable and/or biostable layer as a diffusion barrier without an active agent, covering the subjacent layer completely and/or partially,
or
e) Substantially complete or incomplete covering of the hemocompatible coating with at least one biodegradable and/or biostable layer containing at least one active agent and at least one additional active agent of the same group or of another group with complementary properties in the same or in a different concentration, covalently and/or adhesively bound, or
f) Substantially complete or incomplete covering of the hemocompatible coating with at least two biodegradable and/or biostable layers containing at least one active agent and at least one additional active agent of the same group or of another group with complementary properties in the same or in a different concentration, covalently and/or adhesively bound, and at least one further biodegradable and/or biostable layer as a diffusion barrier without an active agent, covering the subjacent layer completely and/or partially, or
g) Substantially complete or incomplete covering of the hemocompatible coating with at least two biodegradable and/or biostable layers containing at least one active agent in the same or in a different concentration, covalently and/or adhesively bound, and one further biodegradable and/or biostable layer as a diffusion barrier without an active agent, covering the subjacent layer completely and/or partially, and one layer containing the active agent covering aforesaid layer, consisting of at least one active agent covalently and/or adhesively bound to the subjacent layer.

15. Method according to one of the claims 10 - 14, , **characterized in that** the hemocompatible coatings consist of heparin of native origin as well as regioselectively produced derivatives of different degrees of sulfation and acetylation with a molecular weight ranging from the pentasaccharide responsible for the anti-thrombotic effect to the standard molecular weight of commercially available heparin of ca. 13kD, of heparan sulfates and its derivatives, oligo- and polysaccharides of the glycocalix of erythrocytes, oligosaccharides, polysaccharides, completely desulfated and N-reacetylated heparin, desulfated and N-reacetylated heparin, of N-carboxymethylated and/or partially N-acetylated chitosan, polyacrylic acid polyvinyl pyrrolidone and/or polyetherether ketone, polyethyleneglycol or of mixtures of these substances.

16. Method according to one of the claims 10 - 15, **characterized in that** the hemocompatible first coating is preferably covalently bound to the stent, the biostable layer covers completely or incompletely the first layer and the third hemocompatible coating is preferably covalently bound to the second layer.

## Revendications

1. Stent recouvert d'une couche hémocompatible et au moins d'une autre couche sus-jacente comprenant au moins une substance active antiproliférative, anti-inflammatoire et/ou antithrombotique par couplage covalent et/ou adhésif, **caractérisé en ce que** la couche hémocompatible consiste en des dérivés produits de façon regioséléctive à partir d'héparine ayant des dégrées de sulfatation et d'acylation différentes quant au poids moléculaire du pentasaccharide responsable de l'effet antithrombotique jusqu'au standard de poids moléculaire de l'héparine commerciale de ca. 13 kD, en oligosaccharides et polysaccharides de la glycocalix des érythrocytes, en héparine désulfatée et N-réac(ét)ylée et en chitosane , N-carboxymethylé et/ ou partiellement n-ac(ét)ylé ainsi qu'en des mélanges de ces substances.

2. Stent selon la revendication 1, **caractérisé en ce que** les substance actives sont choisies parmi le groupe comprenant sirolimus, rapamycine, everolimus, pimecrolimus, somatostatine, tacrolimus, roxithromycine, dunaimycine, ascomycine, bafilomycine, erythromycine, midecamycine, josamycine, concanamycine, clarithromycine, troléandomycine, folimycine, cerivastatine, simvastatine, lovastatine, fluvastatine, rosuvastatine, atorvastatine, pravastatine, pitavastatine, vinblastine, vincristine, vindesine, vinorelbine, etoposide, teniposide, nimustine, carmustine, lomustine, cyclophosphamide, 4-hydroxyoxycyclophosphamide, estramustine, melphalan, ifosfamide, trofosfamide, chlorambucil, bendamustine, dacarbazine, busulfan, procarbazine, treosulfan, tremozolomide, thiotépa, daunorubicine, doxorubicine, aclarubicine, épirubicine, mitoxantrone, idarubicine, bléomycine, mitomycine, dactinomycine, méthotrexate, fludarabine-5'-dihydrogénophosphate, cladribine, mercaptopurine, thioguanine, cytarabine, fluorouracil, gemcitabine, capecitabine, docétaxel, carboplatine, cisplatine, oxaliplatine, amsacrine, irinotécan, topotécan, hydroxycarbamide, miltéfosine, pentostatine, aldesleukine, trétinoïne, asparaginase, pegaspargase, anastrozole, exémestane, létrozole, formestane, aminoglutéthimide, adriamycine, azithromycine, spiramycine, cepharanthine, inhibiteur 2w de la prolifération des cellules musculaires lisses, épothilone A et B, mitoxantrone, azathioprine, mycophénolate mofétil, c-myc antisens, b-myc antisens, acide betulinique, camptothécine, lapachol, β-lapachone, podophyllotoxine, bétuline, 2-ethylhydrazide de l'acide podophyllique, molgramostime, peginterféron α-2b, lénograstime, filgrastime, macrogol, dacarbazine, basiliximab, daclizumab, sélectine, inhibiteur de la CETP, cadhérine, inhibiteurs de la cytokine, inhibiteur de la COX-2, NFkB, angiopeptine, ciprofloxacine, camptothécine, fluroblastine, anticorps monoclonales inhibitant la prolifération des cellules musculaires, bFGF-antagonistes, probucol, prostaglandines, 1,11-diméthoxycanthin-6-on, 1-hydroxy-11-méthoxycanthin-6-on, scopolectine, colchicine, NO donneurs, pentaerythrityl tetranitrate, sydnoimines, dérivés de S-Nitroso, tamoxifène, staurosporine, β-estradiol, α-estradiol, estriol, estrone, ethinylestradiol, fosfestrol, medroxy-progestérone, cipionates d'estradiol, benzoates d'estradiol, tranilast, kamebakaurin et autres terpénoïdes utilisés pour le traitement anticancéreux, vérapamil, inhibiteurs de tyrosine kinase, tyrphostine, cyclosporine A, paclitaxel, 6-α-hydroxy-paclitaxel, baccatine, taxotères, oligomères macrocycliques du suboxide de carbone (MCS) et ses dérivés, produits synthétiquement ainsi que provenant de sources natives, mofebutazone, acémetacine, diclofenac, lonazolac, dapson, acide acétique o-carbamoyl-phénoxy, lidocaine, ketoprofène, acide méfénamique, piroxicam, meloxicam, chloroquine phosphate, penicillamine, hydroxychloroquine, auranofine, aurothiomalate de sodium, oxaceprol, célécoxib, ß-sitosterine, ademetionine, myrtecaine, polidocanol, nonivamide, levomenthol, benzocaïne, aescine, ellipticine, D-24851 (Calbiochem), colcemide, cytochalasine A-E, indanocine, nocodazole, protéine S 100, bacitracine, antagonistes du récepteur de la vitronectine, azélastine, stimulateur de la guanidyl cyclase, inhibiteur tissulaire des protéinases métalliques 1 et 2, acides nucléiques libres, acides nucléiques incorporés dans des transmetteurs de virus, fragments de DNA et RNA, inhibiteur activateur plasminogène 1, inhibiteur activateur plasminogène 2, oligonucléotides antisens, inhibiteurs VEGF, IGF-1, substances actives provenant du groupe des antibiotiques comme céfadroxil, céfazoline, céfaclor, céfoxitine, tobramycine, gentamycine, pénicillines, dicloxacilline, oxacilline, sulfonamides, metronidazol, antithrombotiques, argatroban, aspirine, abciximab, antithrombine synthétique, bivalirudine, coumadine, enoxaparine, héparine desulfatée et N-acetylée, activateur tissulaire du plasminogène, récepteur membrane plaquettaire GpIIb/IIIa, anticorps contre l'inhibiteur facteur Xa, héparine, hirudine, r-hirudine, PPACK, protamine, prourokinase, streptokinase, warfarine, urokinase, vasodilateurs, dipyridamol, trapidil, nitroprussides, antagonistes de PDGF, triazolopyrimidine et seramine, inhibiteurs de l'ACE, captopril, cilazapril, lisinopril, enalapril, losartan, inhibiteurs de la thioprotease, prostacycline, vapiprost, interféron α, β et γ, antagonistes de l'histamine, bloqueurs de la sérotonine, inhibiteurs d'apoptose, régulateurs de l'apoptose, p65 NFkB, oligonucleotides antisens Bcl-xL, halofuginone, nifédipine, tocophérol, tranilast, molsidomine, polyphénoles du thé, épicatéchine gallate, épigallocatéchine gallate, acides boswelliques et leurs dérivés, leflunomide, anakinra, etanercept, sulfasalazine, etoposide, dicloxacilline, tetracycline, triamcinolone, mutamycin, procainimide, acide rétinoïque, quinidine, disopyramide, flecainide, propafénone, sotalol, amidorone ; stéroïdes produites en manière naturelle et synthétique, bryophylline A, inotodiol, maquiroside A, ghalakinoside, mansonine, strebloside, hydrocortisone, bétaméthasone, déxaméthason, substances non-stéroïdiens, fénoprofène, ibuprofène, indométhacine, naproxène, phenylbutazone et d'autres agents antiviraux acyclovir, ganciclovir, zidovudine, antimycotiques, clotrimazol, flucytosine, griséofulvine, kétoconazole, miconazole, nystatine, terbinafine ; agents antiprotozoaires chloroquine, mefloquine, quinine, en outre terpénoïdes naturelles comme hippocaesculin, barringtogenol-C21-angelate, 14-déhydro-agrostistachin, agroskerine, agrostistachine, 17-hydroxy-agrostistachine, ovatodiolides, acide 4,7-oxycycloanisomelique, baccharinoides B1, B2, B3 et B7, tubeimoside, bruceanoles A,B et C, bruceantinosides C, yadanziosides N, et P, isodéoxyelephantopine, tomenphantopine A et B, coronarine A, B, C et D, acide ursolique, acide hyptatique A, zéorine, iso-iridogermanal, maytenfoliol, effusantine A, excisanin A et B, longikaurin B, sculponéatin C, kamebaunin, leukamenin A et B, 13,18-déhydro-6-alpha-senecioyloxychaparrin, taxamairin A et B, regenilol, triptolide, cymarine, apocymarine, acide aristolochique, anopterine, hydroxy anopterine, anémonine, proto-anémonine, berbérine, chloride de cheliburine, ciguatoxine, sinococuline, bombrestatine A et B, cudraisoflavone A, curcumine, dihydronitidine, chloride de nitidine, 12-beta-hydroxy-pregnadiène-3,20-dione, bilobol, ginkgol, acide ginkgolique, helenalin, indicin, indicin-N-oxide, lasiocarpine, inotodiol, glycoside 1a, podophyllotoxine, justicidine A et B, larréatin, malloterin, mallotochromanol, isobutyryl-mallotochromanol, maquiroside A, marchantine A, maytansine, lycoridicine, margetine, pancratistatine, liriodenine, bisparthenolidine, oxoushinsunine, aristolactame-All, bisparthenolidine, periplocoside A, ghalakinoside, acide usolique, deoxy-psorospermine, psycorubine, ricine A, sanguinarine, acide de froment de Manwu, méthylsorbifolin, sphatheliachromes, stizophylline, mansonine, strebloside, akagerine, dihydrousambaraensin, hydroxyusambarine, strychnopentamine, strychnophylline, usambarine, usambarensine, berbérine, liriodenine, oxoushinsunine, daphnoretine, lariciresinol, methoxy-lariciresinol, syringaresinol, umbelliferone, afromoson, acétyl-vismione B, déacetyl-vismione A, vismione A et B.

3. Stent selon les revendications antérieures, **caractérisé en ce que** la substance antiproliférative, anti-inflammatoire et/ou antithrombotique est contenu dans une concentration pharmaceutiquement active de 0,0001-10 mg / cm² sur la surface du stent.

4. Stent selon la revendication 1, où la couche hémocompatible est covalemment et/ ou adhésivement liée à la surface du stent.

5. Stent selon les revendications antérieures, **caractérisé en ce que** la couche consiste en deux ou plus couches, où la première couche est située directement sur la surface du stent.

6. Stent selon la revendication 5, **caractérisé en ce que** la première couche consiste en une couche hémocompatible qui est complètement ou incomplètement revêtue avec une couche biodégradable et/ou biostable contenant au moins une substance active selon la revendication 2, liée d'une forme covalente et/ou adhésive.

7. Stent selon la revendication 5 ou 6, **caractérisé en ce que** la seconde couche consiste en une couche non-biodégradable contenant au moins une substance active selon la revendication 2, liée d'une forme covalente et/ou adhésive, où cette couche est essentiellement revêtue avec une couche hémocompatible, en préférence covalemment liée.

8. Stent selon la revendication 6 ou 7, **caractérisé en ce que** polyvalérolactone, poly-ε-décalatone, l'acide poly-lactonique, l'acide polyglycolique, polylactides, polyglycolides, copolymères des polylactides et polyglycolides, poly-ε-caprolactone, l'acide butyrique polyhydroxy, polyhydroxybutyrates, polyhydroxyvalérates, polyhydroxybutyrates-co-valérates, poly(1,4-dioxane-2,3-dione), poly(1,3-dioxane-2-one), poly-para-dioxanone, polyanhydrides, anhydrides de l'acide polymaléique, polyhydroxyméthacrylates, fibrine, polycyanoacrylates, polycaprolactone diméthylacrylates, poly-b-acide maléique, acrylates de polycaprolactonbutyl, polymères séquencés multiples de oligocaprolactondioles et oligodioxanedioles, polymères séquencés multiples de polyesters de PEG et poly(butylène téréphtalate), polypivotolactones, triméthyl carbonates de l'acide polyglycolique, polycaprolactone glycolides, poly(g-ethylglutamate), poly(DTH-imino-carbonate), poly(DTE-co-DT-carbonate), poly(bisphenol A-imino-carbonate), polyorthoesters, acide polyglycolique triméthyl-carbonates, poly-triméthyl-carbonates, polyimino-carbonates, poly(N-vinyle)-pyrrolidone, les alcools de polyvinyle, polyesteramides, polyesters glycolés, polyphosphoesters, polyphosphazenes, poly[p-carboxyphenoxy)propane], acide valérianique polyhydroxy, polyanhydrides, oxyde de polyéthylène-oxyde de propylène, polyuréthanes souples, polyuréthanes ayant un résidu d'acide aminé dans le tronc, polyétheresters, oxyde de polyéthylène, oxalate polyalkène, polyorthoesters ainsi que leurs copolymères, lipides, carraghénanes, fibrinogène, amidon, collagène, polymères à base de protéines, les acides polyaminés, les acides polyaminés synthétiques, zéine, zéine modifiée, polyhydroxyalkanoates, l'acide pectique, l'acide actique, fibrine et caséine modifiées et non modifiées, carboxyméthyl sulfate, albumine, l'acide hyaluronique, sulfates d'héparane, héparine, chondroïtine sulfates, dextrane, β-cyclodextrines, copolymères avec PEG et polypropylène glycol, gomme arabique, guar, gelatine, collagène, collagène-N-hydroxy succinimide, lipides, phospholipides, modifications et copolymères et/ou des mélanges des substances mentionnées ci-dessus peuvent être utilisés.

9. Stent selon la revendication 6, 7 ou 8, **caractérisé en ce que** l'acide polyacrylique et polyacrylate, polyméthylméthacrylate, polybutylméthacrylate, polyacrylamide, polyacrylonitriles, polyamides, polyétheramides, polyéthylène amines, polyimides, polycarbonates, polycarbo-uréthanes, cétones de polyvinyle, halogénides de polyvinyle, halogénides de polyvinylidène, éthers de polyvinyle, polyisobutylènes, composés aromatiques de polivinyle, esters de polyvinyle, pyrrollidones de polyvinyle, polyoxymethylènes, oxyde de polytétraméthylène, polyéthylène, polypropylène, polytétrafluoréthylène, polyuréthanes, polyétheruréthanes, polyétheruréthanes de silicone, polyuréthanes de silicone, polycarbonate-uréthanes de silicone, polyoléfine élastomères, polyisobutylènes, gommes EPDM, fluorosilicones, carboxyméthyl chitosane, cétones de polyarylétheréther, cétones de polyétheréther, polyéthylène téréphtalate, polyvalérates, carboxyméthyl cellulose, cellulose, viscose, triacétates de viscose, nitrates de cellulose, acétates de cellulose, hydroxyethyl cellulose, butyrates de cellulose, acetatebutyrates de cellulose, copolymères d'éthylène-vinyle acétate, polysulfones, résines époxy, résines ABS, gommes EPDM, silicones, polysiloxanes, polydiméthylsiloxanes, les halogènes de polyvinyle et copolymères, éthers de cellulose, triacétates de cellulose, chitosane et copolymères et/ ou des mélanges de ceux-ci sont utilisés comme substances biostables pour la couche biostable.

10. Méthode pour le revêtement hémocompatible, antiprolifératif, anti-inflammatoire et/ou antithrombotique des stents selon la revendication 1, **caractérisée par** les étapes suivantes:
a) la mise à disposition d'un stent pas revêtu
b) l'application d'une couche hémocompatible, en préférence covalemment liée
c) revêtement essentiellement complet de la couche hémocompatible par immersion ou par pulvérisation avec au moins une substance active et/ou par couplage covalent de la substance active sur la couche subjacente,
ou
c') revêtement essentiellement complet ou incomplet de la couche hémocompatible par immersion ou par pulvérisation avec au moins une coche biodégradable et/ou biostable contenant au moins une substance active covalemment et/ou adhésivement liée.

11. Méthode selon la revendication 10, **caractérisée par** les étapes a), b), c') et
d) revêtement essentiellement complet de la couche hémocompatible avec au moins une couche biodégradable et biostable contenant au moins une substance active covalemment et/ou adhésivement liée, et une autre couche biodégradable et/ou biostable comme barrière de diffusion sans une substance active et revêtant la couche subjacente complètement et/ou partiellement.

12. Méthode selon la revendication 10, **caractérisée par** les étapes a), b), et
c') revêtement d'une couche biostable contenant au moins une substance active antiproliférative, anti-inflammatoire et/ou antithrombotique covalemment et/ou adhésivement liée; et
d) revêtement essentiellement complet de la couche biostable avec une couche hémocompatible.

13. Méthode selon la revendication 10, **caractérisée par** les étapes a), b), c') et
d) application d'une substance active ou une combinaison de substances actives, covalemment et/ou adhésivement liée à la couche subjacente,
e) revêtement essentiellement complet de la couche hémocompatible avec au moins une couche biodégradable et/ou biostable contenant au moins une substance active covalemment et/ou adhésivement liée,
f) application d'une substance active ou une combinaison de substances actives, covalemment et/ou adhésivement liée, et d'une autre couche biodégradable et/ou biostable comme barrière de diffusion sans une substance active et revêtant la couche subjacente complètement et/ou partiellement.

14. Méthode selon la revendication 10, **caractérisée par** les étapes a), b), et
c') revêtement essentiellement complet ou incomplet de la couche hémocompatible avec au moins deux couches biodégradables et/ou biostables contenant au moins une substance active covalemment et/ou adhésivement liée dans des concentrations différentes dans chaque couche, ou
d) revêtement essentiellement complet ou incomplet de la couche hémocompatible avec au moins deux couches biodégradables et/ou biostables contenant au moins une substance active covalemment et/ou adhésivement liée dans des concentrations différentes dans chaque couche et au moins une autre couche biodégradable et/ou biostable comme barrière de diffusion sans une substance active et revêtant la couche subjacente complètement et/ou partiellement, ou
e) revêtement essentiellement complet ou incomplet de la couche hémocompatible avec au moins une couche biodégradable et/ou biostable contenant au moins une substance active et en outre au moins une substance active de la même classe ou d'une autre classe de propriétés complémentaires dans une concentration égale ou différente, covalemment et/ou adhésivement liée, ou
f) revêtement essentiellement complet ou incomplet de la couche hémocompatible avec au moins deux couches biodégradables et/ou biostables contenant au moins une substance active et en outre au moins une substance active de la même classe ou d'une autre classe de propriétés complémentaires dans une concentration égale ou différente, covalemment et/ou adhésivement liée, et en outre au moins une couche biodégradable et/ou biostable comme barrière de diffusion sans une substance active et revêtant la couche subjacente complètement et/ou partiellement, ou
g) revêtement essentiellement complet ou incomplet de la couche hémocompatible avec au moins deux couches biodégradables et/ou biostables contenant au moins une substance active dans une concentration égale ou différente, covalemment et/ou adhésivement liée, et en outre au moins une couche biodégradable et/ou biostable comme barrière de diffusion sans une substance active et revêtant la couche subjacente complètement et/ou partiellement, et une couche contenant une substance active covalemment et/ou adhésivement liée à la couche subjacente.

15. Méthode selon une des revendications 10-14, **caractérisé en ce que** les couches hémocompatibles consistent en héparine d'origine native et des dérivés produits de façon regioséléctive ayant des dégrées de sulfatation et d'acylation différentes quant au poids moléculaire du pentasaccharide responsable de l'effet antithrombotique jusqu'au standard de poids moléculaire de l'héparine commerciale de ca. 13 kD , en sulfates d'héparane et ses dérivés, en oligosaccharides et polysaccharides de la glycocalix des érythrocytes, en héparine désulfatée et N-réac(ét)ylée, en chitosane N-carboxymethylé et/ ou partiellement n-ac(ét)ylé, acide polyacrylique, pyrrolidone du polyvinyle et/ou cétone de polyétheréther, polyethylène glycol ainsi qu'en des mélanges de ces substances.

16. Méthode selon une des revendications 10 - 15, **caractérisée en ce que** la première couche hémocompatible est de préférence covalemment liée au stent, la couche biostable revête complètement ou incomplètement la première couche et la troisième couche hémocompatible est de préférence covalemment liée à la seconde couche.
